# EUROPEAN PATENT APPLICATION

(11) **EP 2 489 666 A2**
(43) Date of publication of application: **22.08.2012**
(21) Application number: 12156705.1
(22) Date of filing: 02.12.2008
(51) Int. Cl.: C07D 453/02, A61K 31/439, A61P 13/10

(54) **Chemically and chirally pure solifenacin base and its salts**

(30) Priority: 04.12.2007 IN MU23752007; 04.12.2007 IN MU23772007; 11.12.2007 IN MU24212007; 28.05.2008 IN MU11442008
(62) Divisional of application: 08870050.5
(71) Applicant: Cadila Healthcare Limited, Ahmedabad 380 015, Gujarat (IN)
(72) Inventor: Dave, Mayank, Ghanshyambhai, Ahmedabad 380 015 Gujarat (IN); Pandey, Bipin, Ahmedabad 380 015 Gujarat (IN); Kothari, Himanshu, M., Ahmedabad 380 015 Gujarat (IN); Patel, Pankaj, Ramanbhai, Ahmedabad 380 015 Gujarat (IN)
(74) Representative: Bassil, Nicholas Charles

(57) **Abstract**

The present invention provides improved processes for preparing chemically and chirally pure Solifenacin base. The present invention also provides for a composition comprising of a salt of Solifenacin having at least 98% purity. The present invention also discloses certain new salts of Solifenacin as well as new polymorphic forms of Solifenacin hydrochloride and Solifenacin oxalate, in pure form.

## Description

### FIELD OF INVENTION

The present invention discloses improved processes for preparing chemically and chirally pure Solifenacin base and its salts. Specifically the invention describes new salts of Solifenacin selected from gentisate, citrate, hydrobromide, sulphate, nitrate, phosphate, maleate, methane sulphonate, ethane sulphonate, benzene sulphonate, tosylate, α- ketoglutarate, glutarate, nicotinate, malate 1,5-naphthalene disulfonate and ascorbate salts. The invention also describe the new polymorphic forms of Solifenacin hydrochloride and Solifenacine oxalate. In a preferred embodiment, the salts of the present invention have purity of at least 98 %. The invention also describes a process for obtaining chemically and chirally pure Solifenacin base, through the intermediate formation of the salts.

### BACKGROUND OF THE INVENTION

Solifenacin {1(S)-Phenyl-1,2,3,4-tetrahydroisoquinolin-2-carboxylic acid 3(R)-quinuclidinyl ester or [(3R)-1-azabicyclo[2.2.2]oct-3-yl-(1S)-1-phenyl-3,4-dihydroisoquinoline-2-(1H)-carboxylate]}, also known as YM-905 (in its free base form) has the following structure.

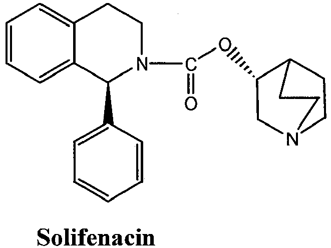

Molecular formula of Solifenacin is C₂₃H₂₆N₂O₂ and its molecular weight is 362.5. Solifenacin and its salts are used as therapeutic agents for Pollakiuria and incontinence of urine due to hyperactive bladder, not as agents for curing hyperactive bladder itself but as therapeutic agents for suppressing the symptoms thereof.

The drug Solifenacin was first disclosed in US. Patent NoS. 6,017,927 and 6,174,896 (CIP of US 6017927) (Yamanouchi Pharmaceuticals). Disclosed therein are compounds with the following general formula

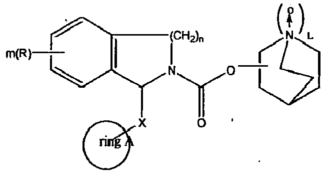

A specific method for producing Solifenacin or its HCl salt is also disclosed, as depicted by the following scheme (Scheme-1).

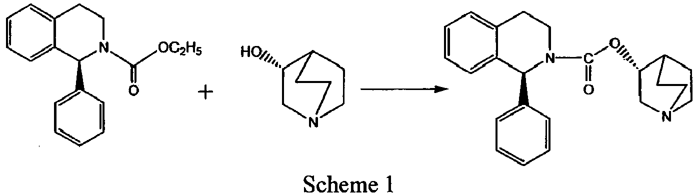

In the reference cited above, the method of preparation of the Solifenacin base using sodium hydride and subsequent conversion of the base to the HCl salt is described, but no data is given for the purity of either the Solifenacin base or the salt. Solifenacin hydrochloride is disclosed particularly in Example-8 of the same patent and crystallization is carried out in a mixture of acetonitrile and diethyl ether. The melting point reported is 212-214 °C. This patent also discloses 3-quinuclidinyl-1-phenyl-1,2,3,4-tetrahydro-2-isoquinoline carboxylate mono oxalate (Example 1) which is the oxalate salt of racemic Solifenacin (M.P. 122-124 °C). The crystallization of the racemate oxalate salt is carried out in a mixture of isopropanol and isopropyl ether.

Polymorphism, the occurrence of different solid state forms, is a property of many molecules and molecular complexes. A single molecular entity may give rise to a variety of solid state forms having distinct crystal structures and physical properties such as melting point, powder X-ray diffraction pattern, infrared (IR) absorption fingerprint and different physicochemical properties. One solid state form may give rise to several polymorphic forms, which are different from one another in all the above properties.

Subsequently, a process for preparation of Solifenacin base and its salts, wherein succinate salt was obtained in high degree of optical purity for medicinal use, was described in EP 1714965 by Astellas Pharma. This document stated that the free base of Solifenacin has the following impurities,

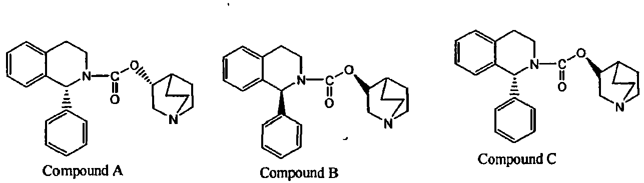

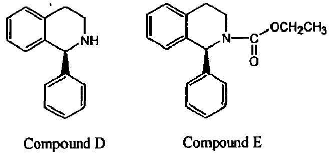

The concentration of the impurities present in the base were as follows:

| | |
|---|---|
| Compound A | 4.51% |
| Compound B | 2.33% |
| Compound C | 0.14% |
| Compound D | 0.32% |
| Compound E | 1.07% |

This document discloses production of Solifenacin hydrochloride and oxalate-containing composition, respectively in reference examples 2 and 4. It states that the hydrochloride and oxalate salts were also not possible to prepare in pure form and only the succinate salt was obtained in a pure form. It also states that the hydrochloride and oxalate containing composition contains the impurities A and B above (A and B both are chiral impurities), at 0.85% or more and 0.50% or more compared to Solifenacin, respectively, even after salt formation and crystallization steps. Thus, there exists a need to prepare both the HCl and oxalate salts as well as other pharmaceutically acceptable salts of Solifenacin in a form that is chemically and chirally pure, is solid and can be handled on an industrial scale. There also exists a need to prepare chemically pure Solifenacin base.

EP 1726304 more specifically discloses the method of preparation of the Solifenacin using an alkoxide base, which makes the process commercially viable. Compared to the process described in US 6,017,927, instead of using sodium hydride having disadvantages like combustion risk and contamination of mineral oil, this method uses an alkoxide base, which overcomes these drawbacks. This document discloses the presence of certain alkylated impurities, which may be present in the Solifenacin base and salts upto 1% concentration.
EP 1757604 discloses four different processes for the preparation of Solifenacin base and the succinate salts.

WO 2008011462 discloses processes for the preparation of Solifenacin base using sodium hydride, and also discloses crystalline form of Solifenacin base and crystalline form of Solifenacin hydrochloride. The salt is prepared as an intermediate step to obtain the succinate salt of Solifenacin in a chemically pure form (purity by HPLC: 99.74%). Nothing is stated about the purity of either the succinate salt or the HCl salt obtained through this process.

WO 2008062282 discloses a process for the preparation of Solifenacin, which is shown below. (Scheme 6).

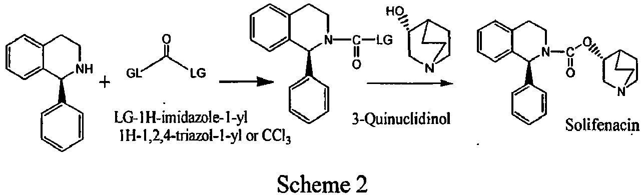

WO 2008077357 application covers process for preparing Solifenacin using non-nucleophilic base. Reaction of crude Solifenacin base with L-tartaric acid provides crystalline Solifenacin hydrogen tartrate salt, which is then transformed to optically pure base as well as other salts (succinate).

WO2008019055 application discloses process for optical resolution of 1-phenyl-1,2,3,4-tetra hydroisoquinoline, which is one of the key intermediate of Solifenacin.

WO2008019103 discloses amorphous and crystalline forms of Solifenacin base as well as process for preparation of the same. In this document they have disclosed form B₁ of Solifenacin base prepared by slurring amorphous Solifenacin base in DIPE solvent.

WO2008013851 discloses amorphous and crystalline forms-I and II of Solifenacin succinate as well as process for preparation of the same.

WO2008120080 disclosed a process wherein 3(R)-quinuclidinol is activated by reaction with bis [1H-1,2,4-triazol-1-yl]-methanone, and the solution obtained is reacted with 1(S)-phenyl-1,2,3,4-tetrahydroisoquinoline to give Solifenacin.

US 20080114029 discloses new polymorphic form of I(S)-phenyl-1,2,3,4-tetrahydroisoquinoline, a key intermediate for the preparation of Solifenacin base.

Though several processes for preparing both the Solifenacin base as well as hydrochloride and oxalate salts are known, very little is said about the chemical purity of any of them. Most of the processes describe the necessity of formation of the succinate salts for improving the chemical purity. Therefore, there is a need to develop a safe modified process for preparing Solifenacin (free base) which give better yields and improved purity. There also is a requirement to prepare such other new salts of Solifenacin, which not only is chemically and chirally pure but also have superior pharmaceutical properties over one or more of the known salts of Solifenacin. We herein disclose an improved process for preparing Solifenacin base in a pure form (chemically and chirally) and also chemically and chirally pure hydrochloride, oxalate, succinate, gentisate, citrate, hydrobromide, sulphate, nitrate, phosphate, maleate, methane sulphonate, ethane sulphonate, benzene sulphonate, tosylate, α- ketoglutarate, glutarate, nicotinate, malate, 1,5-naphthalene disulfonate and ascorbate salts of Solifenacin. In a preferred embodiment, these salts have atleast 98% purity and may be used to prepare the pure Solifenacin base from the impure base through the intermediate formation of any of these salts. Additionally, several of these salts have superior pharmaceutical properties over one or more known salts of Solifenacin.

We herein also disclose new polymorphic forms of Solifenacin hydrochloride and Solifenacin oxalate, in pure form. These forms surprisingly further show certain superior pharmaceutical properties compared to even the succinate salt.

All these salts may be present either in substantially crystalline or amorphous forms or may be present as partially crystalline forms

The new salts of Solifenacin provide a new opportunity to improve the performance of the synthesis the Solifenacin base in a chemically and chirally pure form. Several of these new salts are produced in solid state, having improved characteristics such as stability, flowability and therefore easy to handle in an industrial scale. Some of these salts also have superior biological properties over one or more of the known salts of Solifenacin.

### BRIEF DESCRIPTION OF THE DRAWINGS

FIG. 1 is a powder X-ray diffraction (XRPD) pattern of the Crystalline Solifenacin hydrochloride according to the present invention.
FIG. 2 is a differential scanning calorimetric curve of the Crystalline Solifenacin hydrochloride according to the present invention.

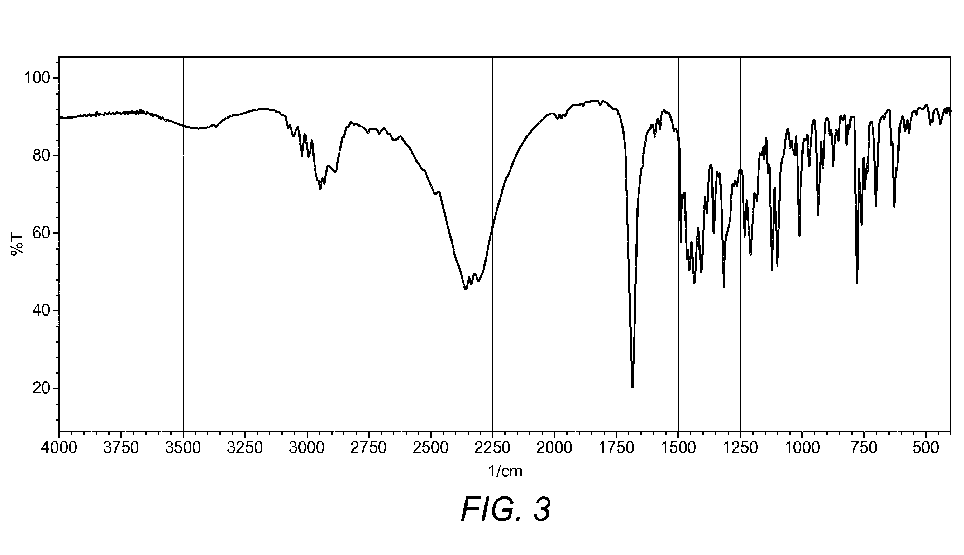

FIG. 3 is a FTIR spectrum of the Crystalline Solifenacin hydrochloride according to the present invention.
FIG. 4 is a powder X-ray diffraction (XRPD) pattern of the Crystalline Solifenacin oxalate according to the present invention.
FIG. 5 is a differential scanning calorimetric curve of the Crystalline Solifenacin oxalate according to the present invention.

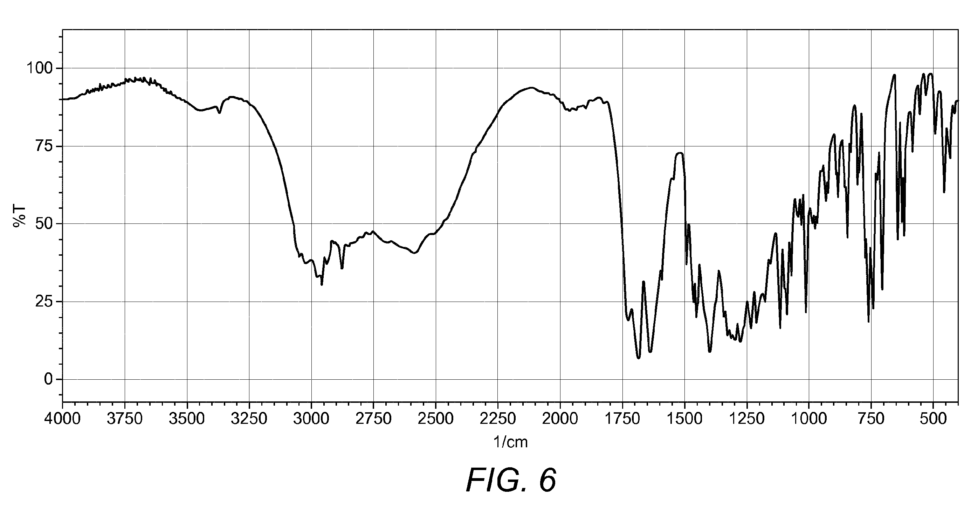

FIG. 6 is a FTIR spectrum of the Crystalline Solifenacin oxalate according to the present invention.
FIG. 7 is a powder X-ray diffraction (XRPD) pattern of the Crystalline Solifenacin gentisate according to the present invention.

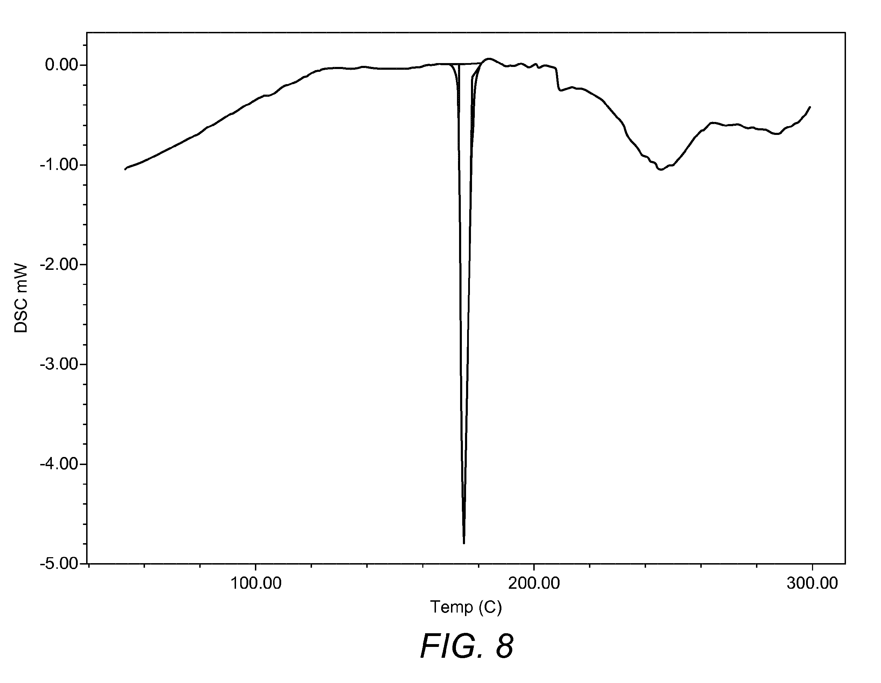

FIG. 8 is a differential scanning calorimetric curve of the Crystalline Solifenacin gentisate according to the present invention

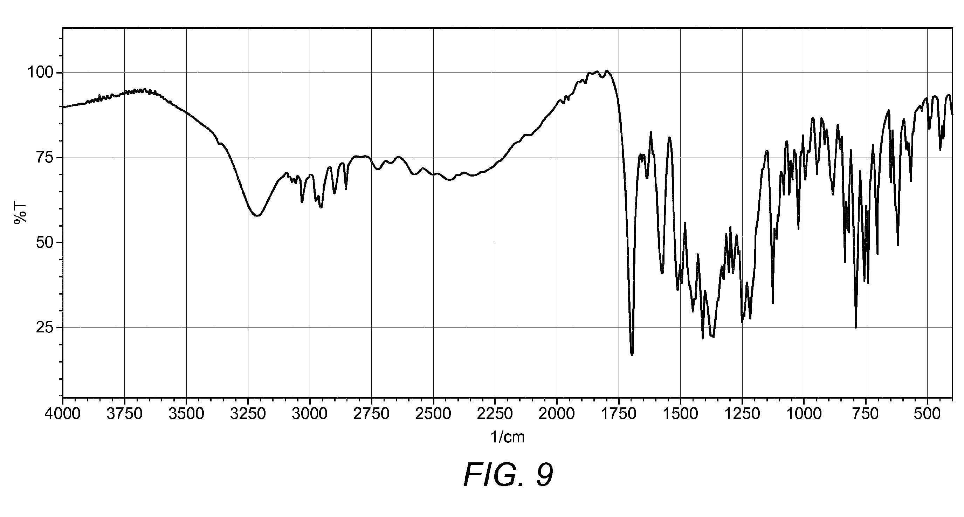

FIG. 9 is a FTIR spectrum of the Crystalline Solifenacin gentisate according to the present invention.
FIG. 10 is a powder X-ray diffraction (XRPD) pattern of the Crystalline Di-paratoluoyl-L-tartaric acid salt of Solifenacin.
FIG. 11 is a powder X-ray diffraction (XRPD) pattern of the Solifenacin besylate according to the present invention.
FIG. 12 is a powder X-ray diffraction (XRPD) pattern of the Solifenacin phosphate according to the present invention.
FIG. 13 is a powder X-ray diffraction (XRPD) pattern of the Solifenacin hydrobromide according to the present invention.
FIG. 14 is a powder X-ray diffraction (XRPD) pattern of the Solifenacin 1,5-naphthalene_disulfonate according to the present invention.
FIG. 15 is a powder X-ray diffraction (XRPD) pattern of the amorphous Solifenacin oxalate according to the present invention.
FIG. 16 is a powder X-ray diffraction (XRPD) pattern of the amorphous Solifenacin gentisate according to the present invention
FIG. 17 is a powder X-ray diffraction (XRPD) pattern of the amorphous Solifenacin hydrochloride according to the present invention.

### OBJECTS OF THE INVENTION

The main objective of the present invention is to provide a composition comprising a salt of Solifenacin having at least 98 % purity.

The composition consisting of a salt of Solifenacin is obtained by a process comprising
(a) an alkali metal salt of 3(R)-Quinuclidinol is reacted with alkyl (S)-1-phenyl-1,2-3,4-tetrahydroisoquinoline-2-carboxylate to obtain a composition comprising the base of Solifenacin and
(b) the composition comprising the base of Solifenacin is converted into the salt by reaction with suitable acid thereby obtaining the composition of the salt, wherein composition has a purity of at least 98 %, based on the weight of the composition.

In an embodiment is provided the compositions of the salts of Solifenacin, wherein the salts are selected from hydrochloride, oxalate, succinate, gentisate, citrate, hydrobromide, sulphate, nitrate, phosphate, maleate, methane sulphonate, ethane sulphonate, benzene sulphonate, tosylate, α- ketoglutarate, glutarate, nicotinate, malate, 1,5-naphthalene disulfonate and ascorbate salts.
In one embodiment is provided an improved process for preparing chemically and/or chirally pure Solifenacin base.
In an embodiment is provided a process for preparing Solifenacin base with ≥ 95 % chiral purity without optical resolution.

In another embodiment is provided a process for further chirally enriching Solifenacin base to about ≥ 99.5% optical purity, by forming diastereoisomeric salts.

In another embodiment is provided a process for purifying Solifenacin base to about ≥ 99.0% chemical and chiral purity through the steps of crystallization and recrystallization, in suitable solvents, without the making of corresponding salt.

In another embodiment is provided a (-)-di-p-toluoyl-L-tartaric acid salt of Solifenacin base.

In a still further embodiment is provided certain pharmaceutically acceptable salts of Solifenacin. In a preferred embodiment, the salts have a purity of at least 98 %.

In an embodiment is provided a process for purifying Solifenacin base by converting corresponding salts of Solifenacin to chemically and chirally pure Solifenacin base having at least 99% purity.

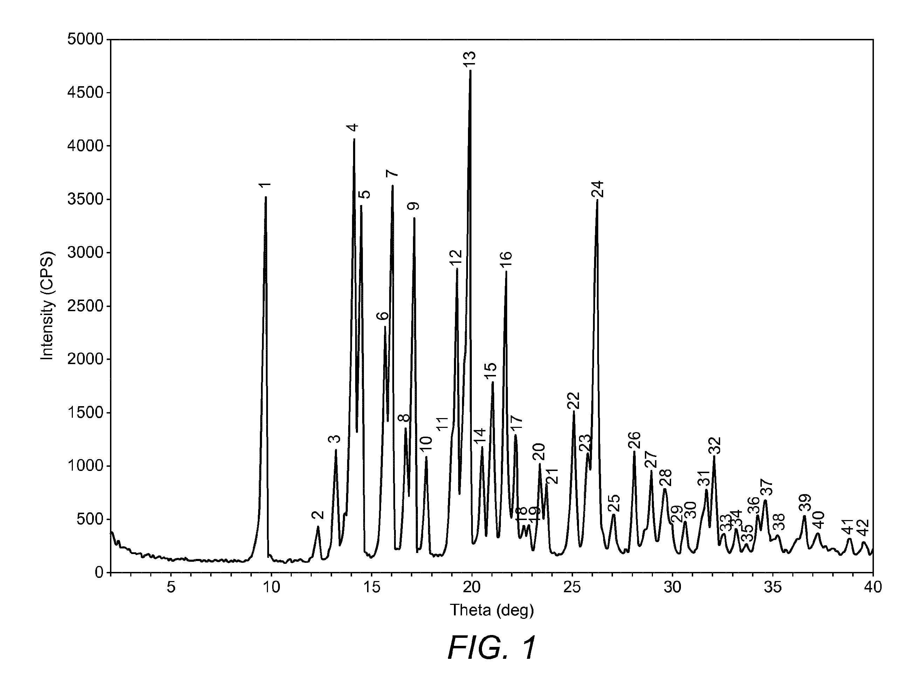

In a still further embodiment is provided pure Solifenacin hydrochloride, characterized by a PXRD pattern substantially as depicted in Figure1.

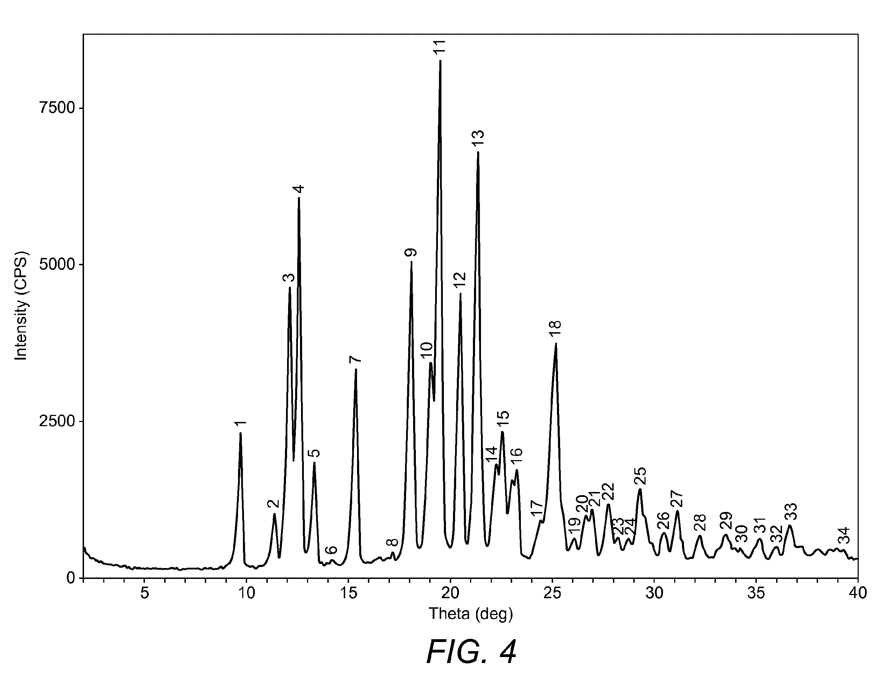

In another embodiment, the invention also disclosed pure Solifenacin oxalate, characterized by a PXRD pattern substantially as depicted in Figure 4.

In a still further embodiment is provided new salts of Solifenacin selected from gentisate, citrate, hydrobromide, sulphate, nitrate, phosphate, maleate, methane sulphonate, ethane sulphonate, benzene sulphonate, tosylate, α-ketoglutarate, glutarate, nicotinate, malate, 1,5-naphthalene disulfonate and ascorbate salts of Solifenacin.

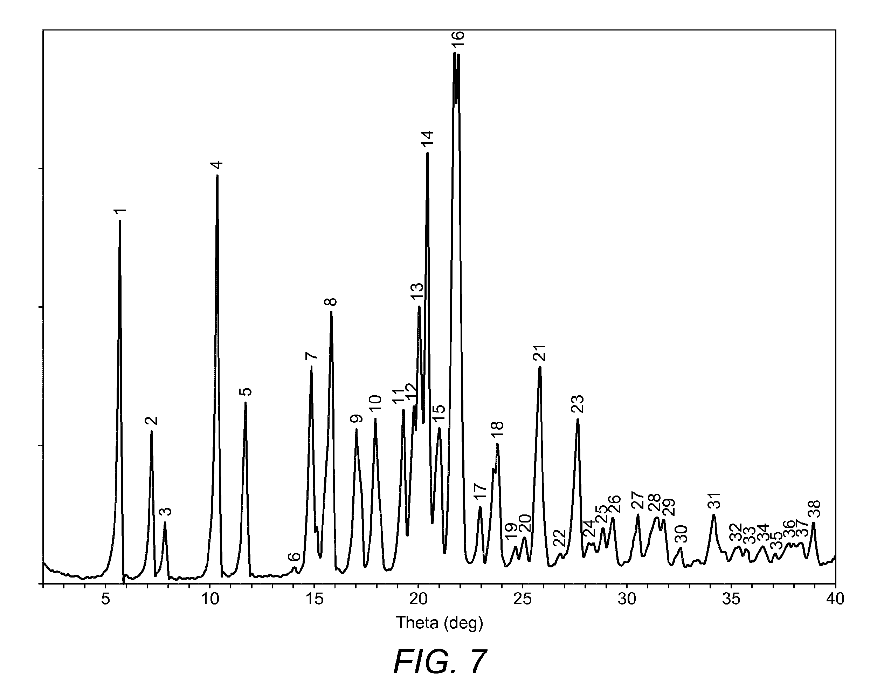

In another embodiment, the invention also disclosed Solifenacin gentisate, characterized by a PXRD pattern substantially as depicted in Figure 7.

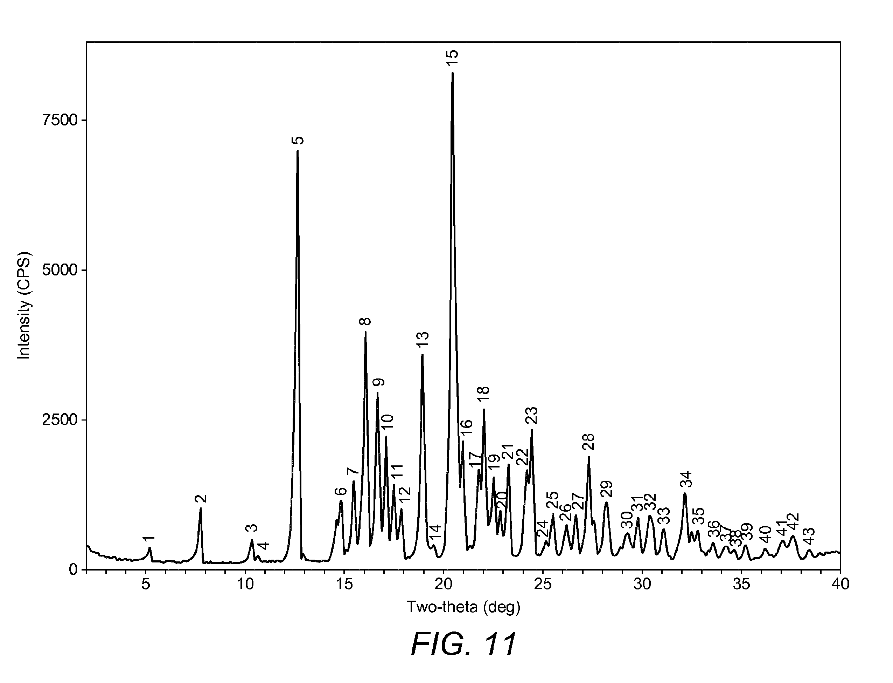

In another embodiment, the invention also disclosed Solifenacin besylate, characterized by a PXRD pattern substantially as depicted in Figure 11.

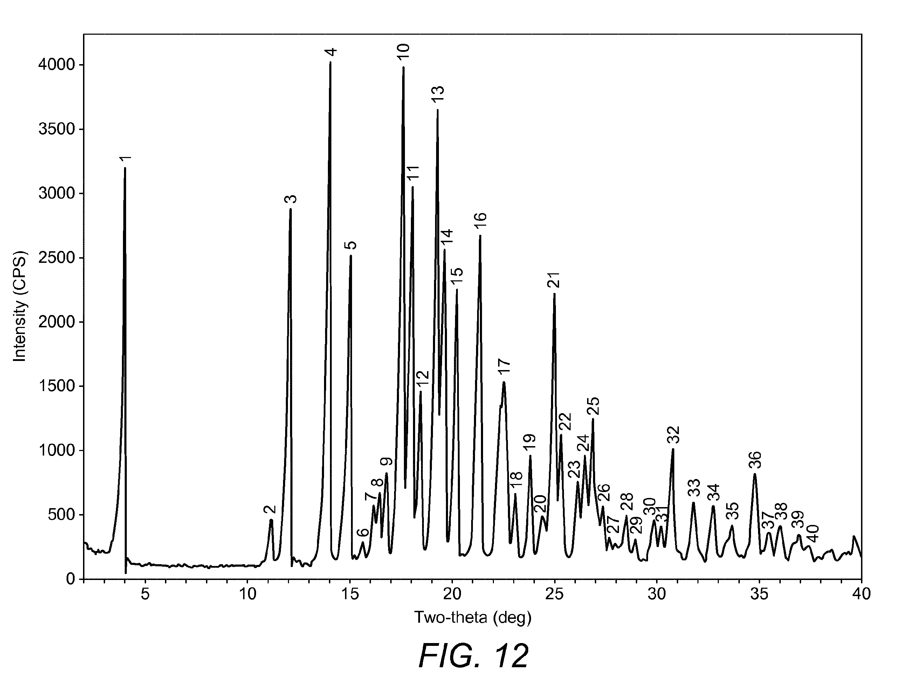

In another embodiment, the invention also disclosed Solifenacin phosphate, characterized by a PXRD pattern substantially as depicted in Figure 12.

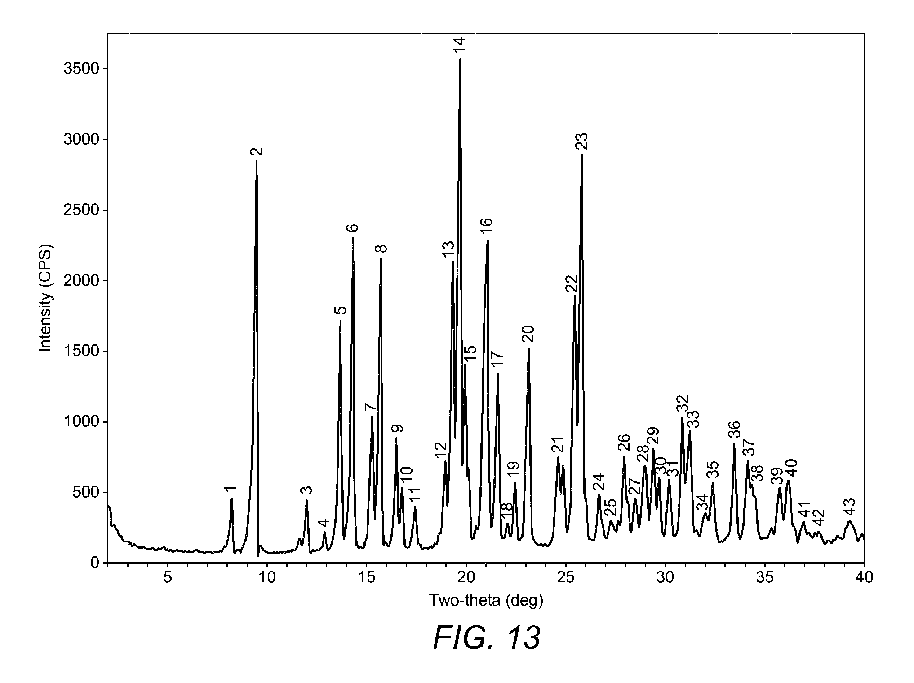

In another embodiment, the invention also disclosed Solifenacin hydrobromide, characterized by a PXRD pattern substantially as depicted in Figure 13.

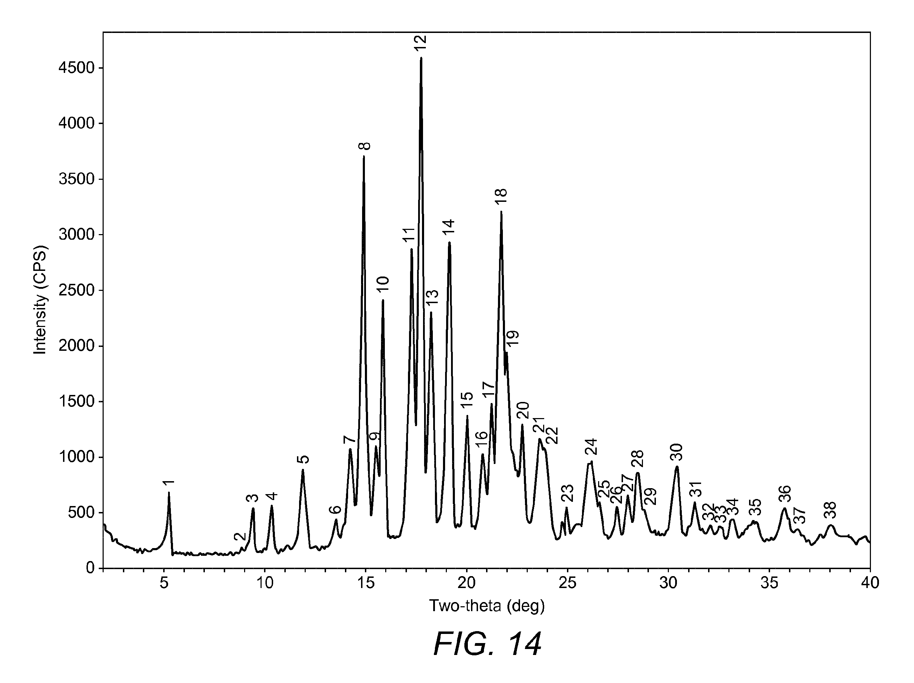

In another embodiment, the invention also disclosed Solifenacin 1,5-Naphthalene disulfonate, characterized by a PXRD pattern substantially as depicted in Figure 14.

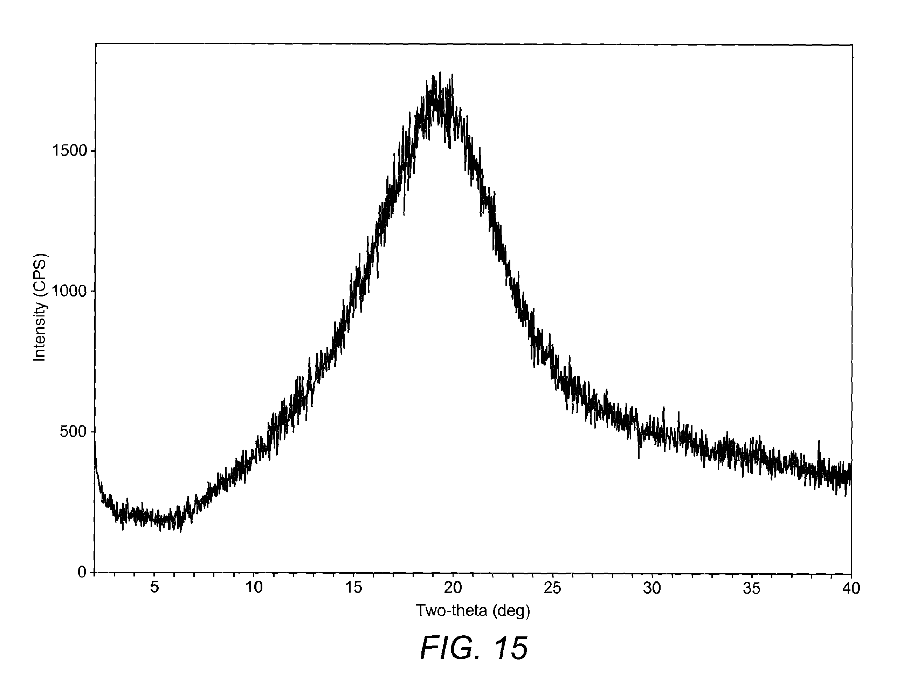

In another embodiment, the invention also disclosed the amorphous Solifenacin oxalate, amorphous Solifenacin gentisate and amorphous Solifenacin hydrochloride was characterized by a PXRD pattern substantially as depicted in Figures 15, 16 and 17 respectively.

In another embodiment, the invention also disclosed a process for preparing a pure hydrochloride, oxalate, succinate, gentisate, citrate, hydrobromide, sulphate, nitrate, phosphate, maleate, methane sulphonate, ethane sulphonate, benzene sulphonate, tosylate, α-ketoglutarate, glutarate, nicotinate, malate 1,5-naphthalene disulfonate and ascorbate salts of Solifenacin.

In a still further embodiment is provided a process for preparing Solifenacin base in chemically and chirally pure form by converting the impure base to one or more of the salts mentioned above and obtaining the base in a pure form from the salt.

In a still further embodiment is provided a process for preparing Solifenacin succinate in chemically and chirally pure form by converting either the Solifenacin base or the salts of Solifenacin obtained in any of the processes of the present invention.

The above and other embodiments are further described in the following paragraphs.

### DETAILED DESCRIPTION

As used herein, the term "reflux temperature" refers to the boiling point of the .solvent.As used herein, the term "PXRD" refers to powder X-ray diffraction.

As used herein, the term "THF" refers to tetrahydrofuran, the term "DCM" refers to dichloro methane, the term "DMF" refers to dimethyl formamide, the term "DIPE" refers to di-isopropyl ether, the term "MIBK" refers to methyl iso butylketone, the term "MTBE" refers to methyl t-butyl ether, the term "MEK" refers to methyl ethyl ketone.

The present invention provides improved processes for preparing chemically and chirally pure Solifenacin base as outlined in Scheme-3. The present invention also provides for a composition comprising of a salt of Solifenacin having at least 98% purity The composition comprising the salt of Solifenacin is obtained by a process comprising
(a) an alkali metal salt of 3(R)-Quinuclidinol is reacted with alkyl (S)-1-phenyl-1,2-3,4-tetrahydroisoquinoline-2-carboxylate to obtain a composition comprising the base of Solifenacin and
(b) the composition comprising the base of Solifenacin is converted into the salt by reaction with suitable acid thereby obtaining the composition of the salt, wherein composition has a purity of at least 98 %, based on the weight of the composition

The process is further described in details in Scheme-3. The present invention also disclose certain new salts of Solifenacin as well as well as new polymorphic forms of Solifenacin hydrochloride and Solifenacin oxalate, in pure form.

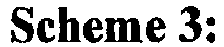

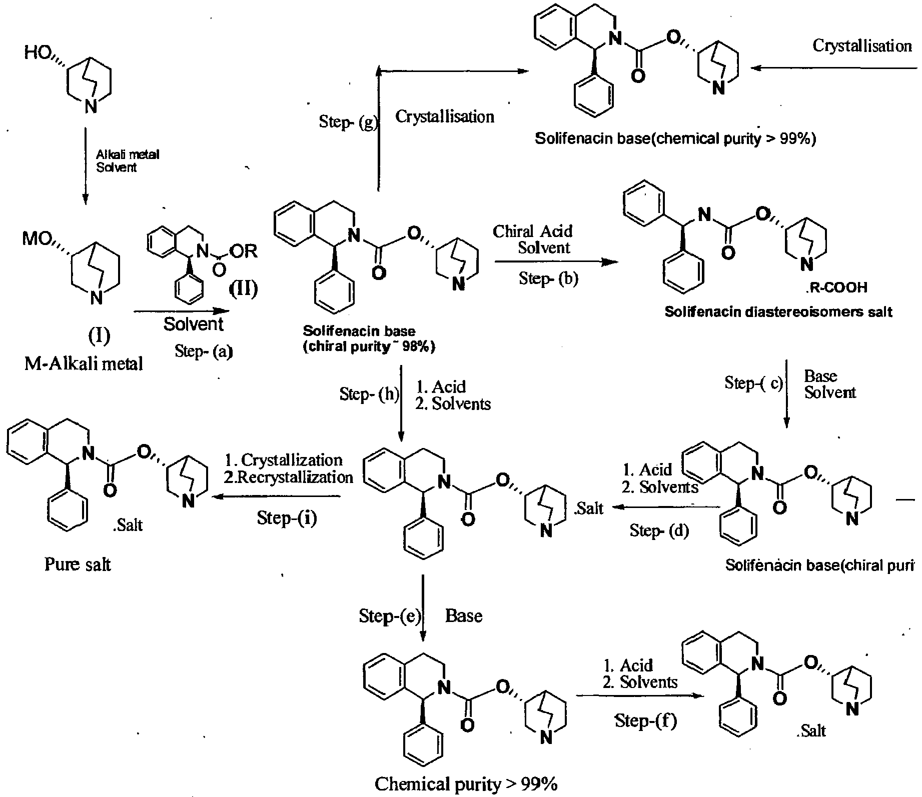

In an embodiment is provided a process for preparing Solifenacin base with ≥ 95 % chiral purity without optical resolution, more preferably with ≥ 98% chiral purity without optical resolution. The process comprises the steps of 1-phenyl-1,2,3,4-tetrahydroisoquinoline was prepared as per method similar to that disclosed in J. Med. Chem. 32, 1242-1248, (1989) and resolution of 1-phenyl-1,2,3,4-tetrahydroisoquinoline was carried out as per similar method disclosed *in* J Med. Chem. 48, 6597-6606, (2005) and Monatsh. Chem. 53-54, 956-962 (1929).

(S)-(+)-1-phenyl-1,2,3,4-tetrahydroisoquinoline was reacted with alkyl chloroformate or alkyl carbonates optionally in the presence of a suitable base in a suitable solvent to obtain Ethyl (S)-1-phenyl-1,2-3,4-tetrahydroisoquinoline-2-carboxylate with retention of configuration.

The suitable base in above step may be suitable organic or inorganic bases which facilitates the amino group of (S)-(+)-1-phenyl-1,2,3,4-tetrahydroisoquinoline to carry out nucleophillic attack. Suitable organic base is selected from C₍₁₋₅₎ alkyl amines, C₍₁₋₅₎ substituted alkyl amines such as triethyl amine (TEA), diisopropyl amine, diisopropylethyl amine, heterocyclic saturated or unsaturated amines, preferably morpholine, piperidine, pyrollidine and pyridine; suitable inorganic bases which can be used is selected from hydroxides such as NaOH, KOH and like, carbonates such as NaHCO₃, Na₂CO₃, K₂CO₃ and like, hydrides such as NaH, n-BuLi, LDA and KHMDS and like or mixtures thereof.

The reaction mixture of above step is maintained at -5 °C to reflux temperature of the solvent used. Preferably, the temperature is at -5 to 30 °C.

The suitable solvent used in above may be selected from suitable hydrocarbons such as benzene, toluene, xylene, ethyl benzene, trimethyl benzene and the like or mixtures thereof, ethers such as tetrahydrofuran, halogenated hydrocarbon solvents such as chloroform, dichloromethane, dichloroethane and the like or mixtures thereof, alcohols such as methanol, ethanol, t-butanol and the like or mixtures thereof, ketones such as acetone, aprotic polar solvent such as DMF, dimethyl acetamide, N-methyl pyrrolidone and water or mixtures thereof. Preferably, it is one or more solvents selected from the group consisting of halogenated hydrocarbons, hydrocarbons, ketones and water. More preferable are halogenated hydrocarbons and ketones; specifically, dichloromethane, acetone and toluene.

The suitable alkyl chloroformate, may be selected from lower alkyl chloroformate such as ethyl chloroformate, isopropyl chloroformate or alkyl carbonates such as diethyl carbonate, t-butyl carbonates. preferably ethyl chloroformate.

The duration of the reaction may be from 15 min. to 5 hrs, more specifically 1 to 3 hrs.

The compound Ethyl (S)-1-phenyl-1,2,3,4-tetrahydroisoquinoline-2-carboxylate of the formula (II) was obtained in a pure form with retention of configuration.

In an embodiment is provided a process for preparing Ethyl (S)-1-phenyl-1,2-3,4-tetrahydroisoquinoline-2-carboxylate (II), wherein suitable solvent used is ketone, preferably acetone.

Separately (R)-3-quinuclidinol was converted to the corresponding metal salt by reacting with suitable alkali metal or metal alkoxide or metal hydride at appropriate temperature in suitable solvent(s), which can be readily determined by a person skilled in the art and will also depend on parameters such as solubility of the metal or metal alkoxide or metal hydride in suitable solvent(s) used and temperature at which minimum racemization can occur.

Suitable alkali metals can be selected from sodium, potassium and lithium, preferably, sodium.

Suitable metal alkoxide can be selected from sodium methoxide, sodium ethoxide, sodium amyloxide, potassium tert. butoxide preferably, sodium methoxide.

Suitable solvent(s) may be selected from suitable hydrocarbons such as benzene, toluene, xylene, ethyl benzene, trimethyl benzene and tetrahydrofuran, halogenated hydrocarbon solvents such as chloroform, dichloromethane, dichloroethane and the like or mixtures thereof, alcohols such as methanol, ethanol, t-butanol and like or mixtures thereof, ketones such as acetone, aprotic solvents such as DMF, dimethyl acetamide or mixtures thereof. Preferably, it is one or more solvents selected from the group consisting of aprotic solvents, hydrocarbons, tetrahydrofuran and ketones. More preferably, suitable solvents may be hydrocarbons or aprotic polar solvent; particularly, toluene and DMF are preferred.

The reaction mixture is maintained at 80 °C to reflux temperature of the solvent used. The duration of the reaction may be from 3 h to 24 hrs, more specifically 3 to 12 hrs.

The metal salt of (R)-3-quinuclidinol was isolated as a solid after removal of solvent at reduced pressure or by filtration. Subsequently, traces of water was removed by Dean Stark apparatus by refluxing in toluene. Care should be taken to ensure complete removal of water.

In one embodiment, as outlined in **step-(a)** in above scheme-3, the metal salt (I) thus obtained was reacted with alkyl (S)-1-phenyl-1,2-3,4-tetrahydroisoquinoline-2-carboxylate (II) in suitable solvents under anhydrous condition to get a composition of Solifenacin base.

Alternatively, The (R)-3-quinuclidinol was converted to the corresponding metal salt by reacting with suitable alkali metal or metal alkoxide or metal hydride in a suitable solvent. The metal salt (I) thus obtained was reacted in situ with Ethyl (S)-1-phenyl-1,2-3,4-tetrahydroisoquinoline-2-carboxylate in a suitable solvent(s) under anhydrous condition to get a composition of Solifenacin base

The suitable solvents used in step-(a) is selected from suitable hydrocarbons such as benzene, toluene, xylene and the like or mixtures thereof, ethers such as tetrahydrofuran and like, alcohols such as methanol, ethanol, t-butanol and like or mixtures thereof, ketones such as acetone, MIBK, aprotic polar solvents such as DMF, dimethyl acetamide or mixtures thereof. Preferably, it is one or more solvents selected from the group consisting of aprotic solvents, hydrocarbons, ethers and ketones. particularly, toluene and DMF are preferred.

In a preferred embodiment, the chiral purity of Ethyl (S)-1-phenyl-1,2,3,4-tetrahydroisoquinoline-2-carboxylate of the formula (II) and (R)-3-quinuclidinol as described above, is above > 99 %, preferably above > 99.5 % more preferably 100 %. The product obtained above was either isolated from the reaction mixture by suitable methods or reacted further as such. The compound (+)-(1S,3'R)-quinuclidin-3'-yl 1-phenyl-1,2,3,4-tetrahydro-isoquinoline-2-carboxylate (Solifenacin base) was obtained with chiral purity > 98 %.

In one of the embodiment, the Solifenacin base obtained is optionally directly converted to the pharmaceutically acceptable salts of Solifenacin. Preferably, the salts have purity of at least 98 %. The pharmaceutically acceptable salts includes the hydrochloride, oxalate, succinate, gentisate, citrate, hydrobromide, sulphate, nitrate, phosphate, maleate, methane sulphonate, ethane sulphonate, benzene sulphonate, tosylate, α- ketoglutarate, glutarate, nicotinate, malate naphthalene-2-sulfonate and ascorbate salts.

Alternatively, the Solifenacin base was converted to its corresponding diastereoisomeric salts as outlined in **step-(b)** of above scheme-3 to enhance the chiral purity of Solifenacin base. The Solifenacin base obtained as per step-(a) having chiral purity > 98 % was treated with suitable chiral acids in suitable solvents to enrich the chiral purity.

Suitable chiral acids used in step-(b) may be selected from (-)-Di-p-toluoyl-L-tartaric acid, di-benzoyl-L-tartaric acid, D-(-)-mandelic acid, L-pyroglutamic acid and (1S)-10-camphorsulfonic acid.

The suitable solvents used in step-(b) may be selected suitable alcohols like methanol, ethanol, isopropanol, butanol, 1,2-dimethoxy ethanol, 2-methoxy ethanol, 2-ethoxy ethanol and ethylene glycol, esters like ethyl acetate and isopropyl acetate, chlorinated solvents like chloroform, dichloromethane, nitriles like acetonitrile, hydrocarbons like toluene, xylene, chlorobenzene, ketones like acetone, methyl ethyl ketone, ethers like diethyl ether, 1,4-dioxane, DIPE, MTBE, THF, aprotic polar solvents such as DMF, DMSO, DMA, water and their suitable mixtures.

The diastereoisomeric salts are purified using suitable solvents selected from but not limited to a lower alkyl chain alcohols such as methanol, ethanol, isopropyl alcohol, esters such as ethyl acetate, iso-propyl acetate, nitriles such as acetonitrile, ketones such as acetone, hydrocarbons such as toluene, xylene or their suitable mixtures.

In one of the preferred embodiment of the present invention the diastereoisomeric salt of the Solifenacin base was prepared by adding (-)-Di-p-toluoyl-L-tartaric acid (DTTA) either as a solid to a solution of Solifenacin base, or after dissolving the acid in a suitable solvent to form a suspension and adding the Solifenacin base in a suitable solvent, at -10 °C to reflux temperature of the solvent used. It is stirred or kept for a period of time as required for a more complete salt formation. The exact time required for complete salt formation can be readily determined by a person skilled in the art. The salt is filtered and washed with a suitable solvent.

In a further embodiment of the invention the diastereoisomeric salt obtained in step-(b) are converted to the Solifenacin base with very high chiral purity as outlined in step-(c) of scheme-3 above. The process involves dissolving the diastereoisomeric salt in a suitable solvent and then it is basified using suitable aqueous solution of base. The free base of the product is extracted in suitable organic solvent. The organic solvent is separated. After water washings and drying, solvent is evaporated to obtained pure Solifenacin base with chiral purity ≥ 99.5% and chemical purity ∼97 %.

Stirring time and volume of the solvent can be readily determined by a person skilled in the art and will also depend on parameters such as solubility of the desired and unwanted stereoisomer's of the salt.

A suitable solvent used in step-(c) is selected from but not limited to esters such as ethyl acetate, iso-propyl acetate and the like, hydrocarbons such as toluene, xylene and the like, ethers such as diethyl ether, 1,4-dioxane, DIPE, MTBE, and the like, halogenated solvents such as chloroform, dichloromethane and the like or their suitable mixtures.

A suitable base used in step-(c) is selected from suitable hydroxides such as NaOH, KOH and like, suitable carbonates such as NaHCO₃, Na₂CO₃, K₂CO₃ and like

In one of the preferred embodiment invention is disclosed the DTTA salt of Solifenacin which was subsequently converted to obtain Solifenacin base having chiral purity > 99.5% and chemical purity of ≥ 97 %.

The invention thus describes the di-paratoluoyl-L-tartaric acid salt of Solifenacin and its use to prepare chirally pure Solifenacin base.

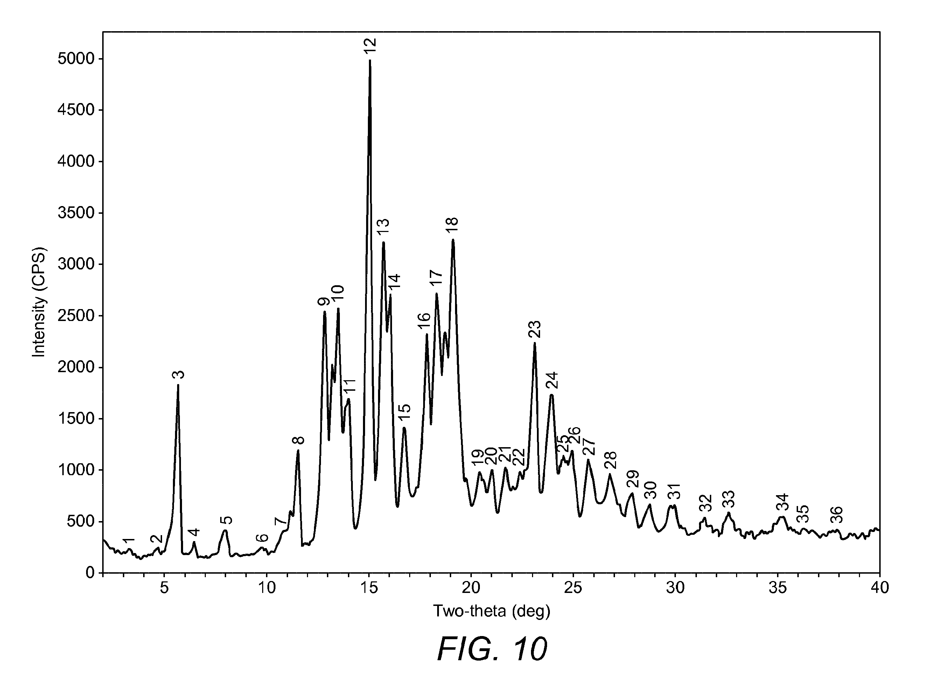

Di-paratoluoyl-L-tartaric acid salt of Solifenacin obtained according to the process of the present invention is characterized by an XPRD pattern substantially in accordance with the pattern Fig.10.

It is also characterized by an XPRD peaks at about 5.67, 11.49, 12.82, 13.49, 13.97, 15.01, 5.68, 16.00, 17.80, 18.28, 19.10, 20.38, 22.36, 23.08, 23.94, 24.50, 24.94 ° ± 2 ° 2θ.

The chirally pure Solifenacin base thus obtained, is converted to its corresponding acid addition salts by reacting with corresponding acids in suitable alcoholic solvents as outlined in **step (d)** of Scheme 3.

The suitable solvents used in step-(d) is selected from alcohols like methanol, ethanol, isopropanol, butanol, 1,2-dimethoxy ethanol, 2-methoxy ethanol, 2-ethoxy ethanol, ethylene glycol and the like, esters like ethyl acetate, isopropyl acetate and the like, chlorinated solvents like chloroform, dichloromethane and the like, nitriles like acetonitrile and the like, hydrocarbons like toluene, xylene, chlorobenzene and the like, ketones like acetone and the like, ethers like diethyl ether, 1,4-dioxane, DIPE, MTBE, THF and the like, aprotic polar solvents such as DMF, DMSO, DMA and the like, water and suitable mixtures of one or more of the solvents described above.

In one preferred embodiment of the invention is disclosed certain pharmaceutically acceptable salts of Solifenacin. In a preferred embodiment, the salts have purity of at least 98 %. In an embodiment, the salts are prepared according to the process described above. The pharmaceutically acceptable salts includes the hydrochloride, oxalate, succinate, gentisate, citrate, hydrobromide, sulphate, nitrate, phosphate, maleate, methane sulphonate, ethane sulphonate, benzene sulphonate, tosylate, α- ketoglutarate, glutarate, nicotinate, malate 1,5-naphthalene disulfonate and ascorbate salts.

In a further embodiment, the salts of Solifenacin obtained in step-(d) is converted to chemically and chirally pure Solifenacin base having at least 99% purity, by using suitable base in suitable solvent(s) as outlined in **step-(e)** of above scheme-3.

Suitable solvent(s) used in step-(e) is selected from but not limited to esters such as ethyl acetate, iso-propyl acetate and the like, hydrocarbon such as toluene, xylene and the like, ethers such as diethyl ether, 1,4-dioxane, DIPE, MTBE and the like, halogenated solvents such as chloroform, dichloromethane and the like or their suitable mixtures.

The suitable base used in step-(e) is selected from hydroxides such as NaOH, KOH and the like, carbonates such as NaHCO₃, Na₂CO₃, K₂CO₃ and like or their suitable mixtures.

The chemically and chirally pure Solifenacin (chemical purity atleast 99% and chiral purity at least 99.5%) base may further be converted to pharmaceutically acceptable salts of Solifenacin by reacting with corresponding acids in suitable solvents as in **step-(f)** of scheme-3.

The suitable solvents used in step-(**f**) is selected from alcohols like methanol, ethanol, isopropanol, butanol, 1,2-dimethoxy ethanol, 2-methoxy ethanol, 2-ethoxy ethanol and ethylene glycol and the like, esters like ethyl acetate and isopropyl acetate and the like, chlorinated solvents like chloroform, dichloromethane and the like, nitriles like acetonitrile and the like, hydrocarbons like toluene, xylene, chlorobenzene and the like, ketones like acetone and the like, ethers like diethyl ether, 1,4-dioxane, DIPE, MTBE, THF and the like, aprotic polar solvents such as DMF, DMSO, DMA and the like, water and suitable mixtures of one or more of the solvents described above.

The pharmaceutically acceptable salt of Solifenacin prepared according to the present invention, preferably having purity at least 98%, more preferably atleast 99%, are selected from hydrochloride, oxalate, succinate, gentisate, citrate, hydrobromide, sulphate, nitrate, phosphate, maleate, methane sulphonate, ethane sulphonate, benzene sulphonate, tosylate, α- ketoglutarate, glutarate, nicotinate, malate 1,5-naphthalene disulfonate and ascorbate salts.

Solifenacin base obtained as above, after steps (a) or (c) may be purified as outlined in **step-(g)** of scheme-3, to get chemically and chiraly pure Solifenacin base. The (+)-(1S,3'R)-quinuclidin-3'-yl1-phenyl-1,2,3,4-tetrahydro-isoquinoline-2-carboxylate (Solifenacin base) obtained above as per step-(a) or (c) was crystallized or purified in suitable solvent. For crystallization, the solution can be prepared at an elevated temperature, if desired, to achieve a desired concentration. Any temperature is acceptable for the dissolution as long as a clear solution of the compound is obtained and is not detrimental to the compound chemically or physically. The exact temperature required can be readily determined by a person skilled in the art and will also depend on parameter such as concentration. The solution may be brought down to -10 °C to 30 °C for further processing/precipitation, if required, otherwise an elevated temperature may be used and stirred or kept for a period of time as required for a more complete isolation of the product. The exact cooling temperature and time required for complete isolation can be readily determined by a person skilled in the art and will depend on parameters such as concentration and temperature of the solution or slurry.

For slurring or tituration temperature, time and volume of the solvent can be readily determined by a person skilled in the art and will also depend on parameters such as solubility of the compound and solubility of impurities in suitable solvent(s)

The suitable solvent used in step-(g) is selected from C(₁₋₆₎ alkyl alcohols, 2-methyl 1,2-dimethoxy ethanol, 2-methoxy ethanol, 2-ethoxy ethanol, ethylene glycol and the like esters such as methyl acetate, ethyl acetate, propyl acetate, butyl acetate, isopropyl acetate, isobutyl acetate, ethyl formate and the like, nitriles such as acetonitrile and the like, ketones such as acetone, methyl ethyl ketone, methyl isobutyl ketone and the like, hydrocarbons such as toluene, xylene, cumene, chlorobenzene, tetralin, pentane, hexane, heptane, cyclohexane, methyl cyclohexane and the like, water, ethers such as dimethyl formamide, dimethylsulfoxide, dimethyl acetamide, dimethyl ether, diethyl ether, diisopropyl ether, t-butyl methyl ether, cyclopentyl methyl ether, phenyl methyl ether, diphenyl ether, tetrahydrofuran, and the like, formamide, N-methyl pyrrolidone, haloginated solvent such as dichloromethane, dichloroethane, chloroform, 1,1,1-trichloro ethene, 1,1,1-trichloro ethane and the like or their suitable mixtures to obtain chemically and chirally pure solid Solifenacin base.

The chemically and chirally pure Solifenacin base obtained in step-g may subsequently be converted to the corresponding chemically and chirally pure pharmaceutically acceptable salt by reacting with corresponding acid in suitable solvents as described above.

Crystallization of the acid addition salts of Solifenacin, obtained as per step-d or h above, by a process as outlined in **step-(i)** of scheme-3, provides for chemically (>99%) and chirally (>99%) pure salts of Solifenacin.

The crystallization is carried out in suitable solvents such as alcohols, esters, chlorinated solvents like chloroform, dichloromethane, nitriles like acetonitrile, hydrocarbons like hexane, heptane, cyclohexane, toluene, xylene, chloro benzene, ketones like acetone, ethers like diethyl ether, 1,4-dioxane, DIPE, MTBE, THF and DMF, DMSO, DMA, Formamide, NMP, 1,2-dimethoxy ethanol,2-methoxy ethanol, 2-ethoxy ethanol, ethylene glycol, water or their suitable mixtures, and subsequently recrystallized from suitable solvents selected from any of the above.

It has surprisingly been found according to the invention that, some of the pharmaceutically acceptable salt of Solifenacin obtained were 99.9% pure, such as the oxalate, hydrochloride, gentisate, phosphate, besylate and hydrobromide salts of Solifenacin.

It has also been found that, by the process of the present invention, pharmaceutically acceptable salts of Solifenacin can be prepared having a residual solvent (as measured in terms of content by weight of the pharmaceutically acceptable salt) of Solifenacin are under 1%, preferably under 0.5% and more preferably under 0.05% by GC-FID as shown in table 1.

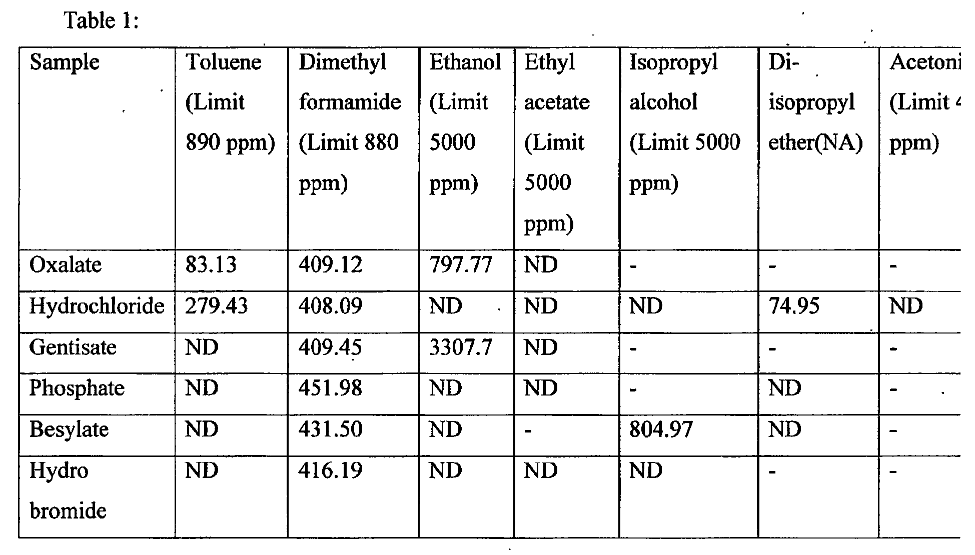

The pharmaceutically acceptable salt of Solifenacin may be present either in substantially crystalline or substantially amorphous form or may be present in mixtures of crystalline and amorphous form which were characterized by PXRD peaks.

It has additionally been found according to the invention that the desired and, in particular, crystalline form of salts of Solifenacin can be prepared simply and in forms which are favorable for further processing to pharmaceutical formulation.

These salts were further characterized by PXRD peaks and DSC values.

### Analytical processes:

The complete x-ray powder spectrum, was recorded with a Rigaku D/Max 2200 VPC X-ray powder diffractometer model using copper radiation. The X-ray diffraction pattern was recorded by keeping the instrument parameters as below: X-ray: Cu/40kv/30mA, Diverging slit: 1°, Scattering slit : 1°, Receiving slit : 0.15 mm, Monochromator RS : 0.8 mm, Counter : Scintillation counter,

Scan mode: Continuous, Scan speed: 3.000°/min., Sampling width: 0.020°, Scan axes: 2 theta vs CPS, Scan range: 2° to 40.0°, Theta offset: 0.000

Differential scanning calorimetric analysis was carried out in a DSC-60 model from Shimadzu (S/W : TA-60WS Aquisition version 2.1.0.0) by keeping following parameters,
Sample Size : Approx. 1-2mg, Sample Pans : Hermetic/Crimping Pans,
Start Temperature : 50°C, End Temperature : 300°C, Rate of Heating : 10 °C/min.,
Purge Gas : Nitrogen, Flowrate : 20 ml/min

The infrared (IR) spectrum has been recorded on a Shimadzu FTIR-8400 model spectrophotometer, between 450 cm⁻¹ and 4000 cm⁻¹, with a resolution of 4 cm⁻¹ in a KBr pellet.

Solifenacin base and its salts were analyzed for purity by analytical HPLC at λₘₐₓ 220nm using column J' sphere-ODS, 150mm x 4.6mm x 4µ or its equivalent on AGILENT 1100 series under the following conditions,
Detector : UV absorption photometer Wave length : 220 nm Column temp. : 30 °C
Flow rate : 1.0 mL/min. Injection Vol. : 5 µL
Mobile Phase (gradient elution) : 0.05 TFA buffer in water:Acetonitrile

Solifenacin base and its salts were analyzed for chiral purity by analytical HPLC at λₘₐₓ 220 nm using column Chiral-Cel OD, 250mm x 4.6mm x 5µ or its equivalent on Shimadzu LCVP model under the following conditions,
Detector : UV absorption photometer Wave length : 220 nm Column temp. : 30 °C
Flow rate : 0.8 mL/min. Injection Vol. : 5 µL
Mobile Phase : n-Hexane: IPA: Diethylamine (90:10:0.1)
Melting points were taken on VEEGO make model VMP-D melting point apparatus and are uncorrected.

A new polymorphic form of Solifenacin hydrochloride obtained according to the process and of the present invention is characterized by an XPRD pattern substantially in accordance with the pattern Fig. 1. Solifenacin hydrochloride obtained is also characterized by an XPRD peaks at about 9.61, 13.18, 14.02, 14.39, 15.58, 15.89, 17.0, 18.94,19.18, 19.78, 20.98, 21.61 and 26.12 ° ± 0.2° degrees 2θ and has melting point in the range of 260-263 °C.

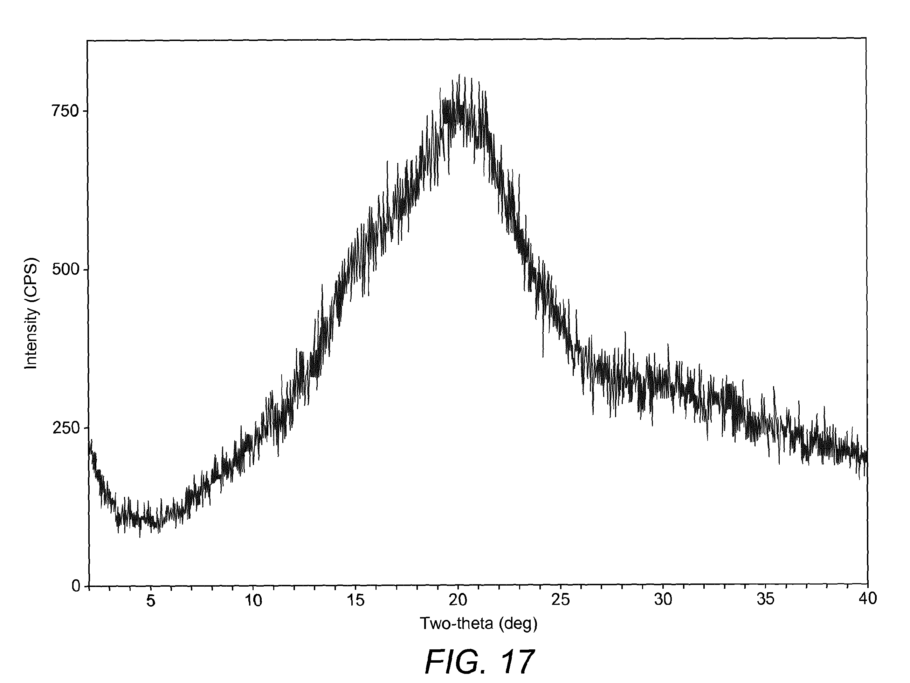

The present invention also discloses amorphous form of Solifenacin hydrochloride characterized by an XPRD pattern substantially in accordance with the pattern Fig. 17.

A new polymorphic form of Solifenacin oxalate obtained according to the process of the present invention is characterized by an XPRD pattern substantially in accordance with the pattern Fig. 4. Solifenacin oxalate obtained is also characterized by an XPRD peaks at about 9.78, 12.23, 12.68, 13.42, 15.44, 18.18, 19.56, 20.58, 21.45 and 25.24 *°* ± 0.2 ° degrees 2θ and has melting point in between the range of 169-174 °C.

The present invention also discloses amorphous form of Solifenacin oxalate characterized by an XPRD pattern substantially in accordance with the pattern Fig. 15.

In one of the preferred embodiment, the invention also discloses new salts of Solifenacin, which are selected from gentisate, citrate, hydrobromide, sulphate, nitrate, phosphate, maleate, methane sulphonate, ethane sulphonate, benzene sulphonate, tosylate, α- ketoglutarate, glutarate, nicotinate, malate naphthalene-2-sulfonate and ascorbate salts.

Solifenacin gentisate obtained according to the process of the present invention is further characterized by an XPRD pattern substantially in accordance with the pattern Fig. 7.

Solifenacin gentisate obtained is also characterized by an XPRD peaks at about 5.56, 7.06, 7.70, 10.15, 14.63, 15.54, 17.63, 19.4, 19.7, 20.08, 20.68, 21.58, 25.48 ° ± 0.2 ° degrees 2θ and has melting point in the range of 164-174 °C.

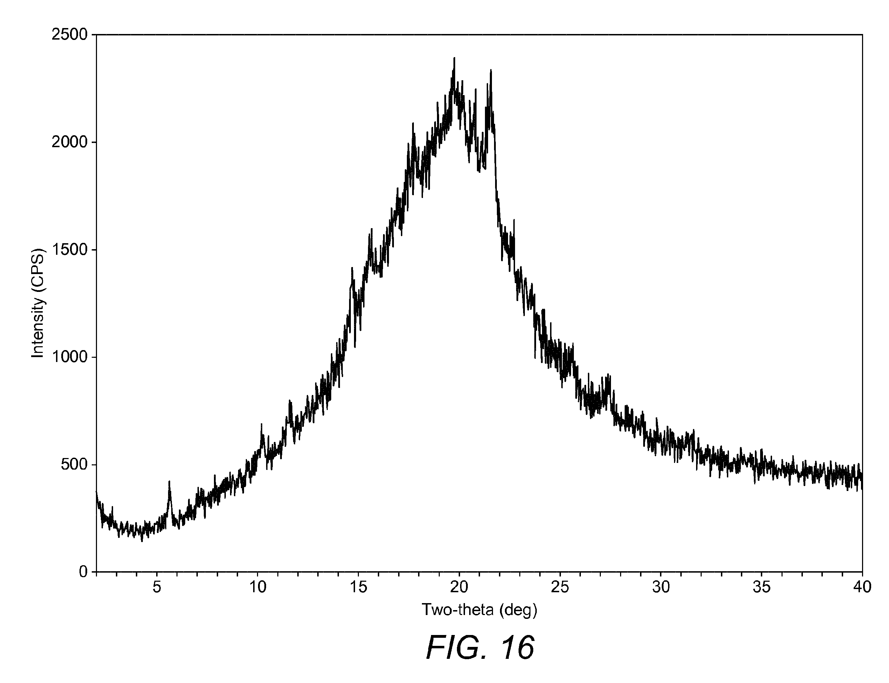

The present invention also discloses amorphous form of Solifenacin gentisate, characterized by an XPRD pattern substantially in accordance with the pattern Fig. 16. Solifenacin besylate obtained according to the present invention is characterized by an XPRD pattern substantially in accordance with the pattern Fig. 11.

Solifenacin besylate obtained is also characterized by an XPRD peaks at about 5.14, 7.69, 10.27, 12.58, 14.76, 16.05, 16.66, 17.08, 17.46, 18.92, 20.46, 20.93, 21.74, 22.02, 23.22, 24.13, 24.39, 27.28, 28.18° ± 0.2° degrees 2θ and has melting point in the range of 191-194 °C.

Solifenacin phosphate obtained according to the present invention is characterized by an XPRD pattern substantially in accordance with the pattern Fig. 12.

Solifenacin phosphate obtained according to the present invention is also characterized by an XPRD peaks at about 3.99, 11.22, 12.07, 13.96, 14.96, 16.15, 16.42, 17.51, 17.96, 18.43,19.2, 19.58, 20.2, 21.31, 22.53, 23.83, 24.94, 25.29, 26.10, 26.46, 26.82, 28.43, 29.74 ° ± 0.2 degrees (2θ) and has melting point in the range of 225-245 °C.

Solifenacin hydrobromide obtained according to the present invention is characterized by an XPRD pattern substantially in accordance with the pattern Fig. 13.

Solifenacin hydrobromide obtained is also characterized by an XPRD peaks at about 8.2, 9.4, 11.98, 13.68, 14.3, 15.27, 15.69, 16.49, 16.77,18.93, 19.29, 19.62, 19.91, 21.04, 21.58, 22.46, 23.14, 24.64, 25.44, 25.78, 27.94, 28.98, 29.43, 30.88, 31.24, 32.38, 33.48, 34.16, 34.43 o ± 0.2 ° degrees (2θ) and has melting point in the range of 235-240 °C.

Solifenacin 1,5-Naphthalene disulfonate obtained according to the process of the present invention is characterized by an XPRD pattern substantially in accordance with the pattern Fig. 14.

Solifenacin 1,5-Naphthalene disulfonate obtained is also characterized by an XPRD peaks at about 5.22, 9.33, 10.28, 11.84, 14.16, 14.84, 15.42, 15.78, 17.18, 17.65, 18.16, 19.06, 19.96, 21.12, 21.63, 21.92, 23.55, 23.80, 26.02, 28.4, 30.35° degrees 2θ and has melting point in the range of 153-160 °C.

The crystalline form of Solifenacin oxalate prepared according to the present invention will be designated hereinafter as "Crystalline Form II of Solifenacin oxalate"

The crystalline form of solifenacin hydrochloride prepared according to the present invention will be designated hereinafter as "Crystalline Form II of Solifenacin hydrochloride"

Several of the Solifenacin salts prepared according to the present invention has chemical and polymorphic stability on storage. Results of the stability of Solifenacin hydrochloride, Solifenacin oxalate and Solifenacin gentisate are shown in table 2.

**Table-2: STABILITY STUDY ON STORAGE (40 °C ± 2 °C / 75% ± 5% RH)**

| **Solifenacin Salt** | **Test** | | **Initial** | **After 3 Month** |
|---|---|---|---|---|
| **Solifenacin Gentisate** | Description | | Off white colored powder | Off white colored powder |
| | Purity By HPLC) | | 99.63 % | 99.46 % |
| | Water (by KFR) | | 0.16 % | 0.29 % |
| | Polymorphism | XRD | Crystalline pattern | No Change |
| **Solifenacin Hydrochloride** | Description | | Off white colored powder | Off white colored powder |
| | Purity (By HPLC) | | 99.54 % | 99.6% |
| | Water (by KFR) | | 0.14 % | 0.32 % |
| | Polymorphism | XRD | Crystalline pattern | No Change |
| **Solifenacin Oxalate** | Description | | Off white colored powder | Off white colored powder |
| | Purity (By HPLC) | | 99.49 % | 99.36 % |
| | Water (by KFR) | | 0.18 % | 0.33 % |
| | Polymorphism | XRD | Crystalline pattern | No Change |

### Study of Pharmacokinetic properties of the new salts of Solifenacin:

Results of the pharmacokinetic profile of some of the new salts of Solifenacin prepared according to the present invention are shown in tables 3a and 3b.

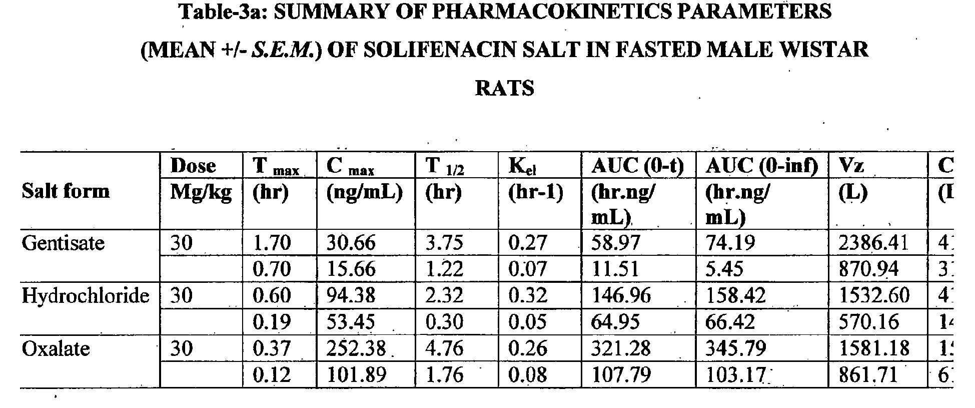

**Table-3b: SUMMARY OF PHARMACOKINETICS PARAMETERS (MEAN +/- S.E.M.) OF SOLIFENACIN SALT IN FASTED MALE WISTAR RATS**

| **Sr.No.** | **Salt Form** | **Dose** | **No of animals** | **Tₘₐₓ** | **Cₘₐₓ** | **T_{1/2}** | **Kₑₗ** | **AUC (0-t)** | **AUC (0-inf)** |
|---|---|---|---|---|---|---|---|---|---|
| | | **mg/kg** | | **(hr)** | **(ng/ml)** | **(hr)** | **(hr-1)** | **(hr.ng/ml)** | **(hr.ng/ml)** |
| 1 | Solifenacin | 30 | 5 | **1.73** | **12.84** | **1.95** | **0.36** | **45.54** | **49.27** |
| | Besylate | | | 0.27 | 2.15 | 0.15 | 0.02 | 3.68 | 3.55 |
| 2 | Solifenacin | 30 | 5 | **1.00** | **12.63** | **2.43** | **0.29** | **50.58** | **57.63** |
| | Phosphate | | | 0.42 | 1.63 | 0.10 | 0.01 | 4.55 | 5.06 |
| 3 | Solifenacin | 30 | 3 out of 5 | **2.00** | **10.29** | **2.29** | **0.30** | **47.24** | **53.27** |
| | Citrate | | | 0.00 | 0.41 | 0.08 | 0.01 | 3.40 | 4.19 |
| 4 | Solifenacin | 30 | 5 | **1.27** | **14.12** | **3.36** | **0.23** | **59.93** | **73.99** |
| | Hydrobromide | | | 0.32 | 2.81 | 0.47 | 0.04 | 5.55 | 4.84 |
| 5 | Solifenacin | 30 | 5 | **1.30** | **29.04** | **2.57** | **0.29** | **85.66** | **96.99** |
| | Ascorbate | | | 0.43 | 11.61 | 0.32 | 0.05 | 19.02 | 18.17 |
| 6 | Solifenacin | 30 | 5 | **2.03** | **26.61** | **2.79** | **0.26** | **47.42** | **54.77** |
| | Tosylate | | | 0.61 | 17.70 | 0.34 | 0.03 | 11.65 | 12.11 |
| 7 | Solifenacin | 30 | 7 | **1.62** | **9.10** | **6.19** | **0.18** | **49.77** | **78.78** |
| | Oxalate | | | 0.62 | 1.33 | 1.79 | 0.04 | 7.34 | 13.24 |
| 8 | Solifenacin | 30 | 7 | **1.05** | **13.67** | **3.65** | **0.23** | **50.54** | **59.52** |
| | Hydrochloride | | | 0.25 | 1.74 | 0.83 | 0.03 | 6.92 | 8.30 |
| 9 | Solifenacin | 30 | 5 | **1.70** | **30.66** | **3.75** | **0.27** | **58.97** | **74.19** |
| | Gentisate | | | 0.70 | 15.66 | 1.22 | 0.07 | 11.51 | 5.45 |
| 10 | Solifenacin | 30 | 5 | **2.67** | **23.72** | **4.34** | **0.20** | **60.09** | **76.69** |
| | Succinate | | | 1.50 | 9.98 | 1.15 | 0.04 | 19.20 | 16.56 |

The Solifenacin oxalate salt showed rapid absorption and ∼10 fold higher plasma concentration (Bio available, Cmax) and 5 times higher plasma exposure compared to Solifenacin succinate salt. The oxalate salt also showed higher T1/2 value compared to the succinate salt.

The HCl salt also showed better PK profile and ∼ 3 times higher plasma exposure as compared to succinate.

Solifenacin phosphate, bromide, and hydrochloride showed faster absorption (T max : ∼1.0 -1.3hr) as compared to Solifenacin succinate.
Solifenacin besylate, citrate, tosylate, oxalate and gentisate salt showed moderately faster absorption (Tmax: 1.6 to 2.0 hrs) as compared to Solifenacin succinate (Tmax: 6.67 hr)

The Gentisate salt had pharmacokinetic profile comparable with Solifenacin succinate salt and peak plasma concentrations of gentisate and tosylate salt were comparable with comparative bioavailability of Solifenacin succinate.

Solifenacin ascorbate showed 20% better absorption and rate of absorption was faster than the succinate salt.

### Study of Intrinsic dissolution rate of different salts of Solifenacin:

Some of the new salts showed comparable or better intrinsic dissolution rate than the succinate salt of Solifenacin. The results are shown.in table-4.

**Table-4: INTRINSIC DISSOLUTION RATE OF VARIOUS SALTS OF SOLIFENACIN**

| **Sr. No.** | **Solifenacin Salt** | **Intrinsic dissolution rate(mg/min.cm²)** | | |
|---|---|---|---|---|
| | | **pH 1.2 HCl** | **pH 4.5 Acetate Buffer** | **pH 6.8 Phosphate Buffer** |
| 1. | Gentisate | 6.845 | 0.2356 | 0.4932 |
| 2. | Succinate | 10.666 | 9.845 | 11.002 |
| 3. | Oxalate | 6.5822 | 6.5084 | 4.9096 |
| 4. | HCl | 6.6836 | 21.08 | 20.262 |

In one embodiment of the invention is disclosed a pharmaceutical composition of Solifenacin or its pharmaceutically acceptable salts together with a liquid or solid carrier, excipients as is known in the art wherein the Solifenacin is produced by any one of the above disclosed processes and the pharmaceutically acceptable salts are selected from hydrochloride, oxalate, succinate, gentisate, citrate, hydrobromide, sulphate, nitrate, phosphate, maleate, methane sulphonate, ethane sulphonate, benzene sulphonate, tosylate, α- ketoglutarate, glutarate, nicotinate, malate, 1,5-naphthalene disulfonate and ascorbate salts.

The invention is further exemplified by the following non-limiting examples, which are illustrative representing the preferred modes of carrying out the invention. The invention's scope is not limited to these specific embodiments only but should be read in conjunction with what is disclosed anywhere else in the specification together with those information and knowledge which are within the general understanding of a person skilled in the art.

### Example 1

### Preparation of (+)-(1S,3'R)-quinuclidin-3'-yl 1-phenyl-1,2,3,4-tetrahydroisoquinoline-2-carboxylate (Solifenacin)

To the cooled solution of freshly prepared sodium methoxide (1.8 g), (R)-3-quinuclidinol HCl (6.4 g) was added under N₂ atmosphere. It was stirred at 5-30 °C for 30 min to 1 hrs. Distilled out the solvent at reduced pressure. To the semi-solid mass dry toluene was added. Reaction mixture was heated to reflux temp. and stirred for 1-3 h. During this process, traces of water and methanol were removed azeotropically by using Dean-Stark apparatus and was cooled to 60-70 °C. (S)- Ethyl 1-phenyl-1,2,3,4-tetrahydroisoquinoline-2-caboxylate (10 g) dissolved in dry toluene and dry DMF were added. It was again heated to reflux temperature and stirred for 5-25 h while distilling off solvent to remove ethanol at intervals with addition of fresh quantity of dry solvent. It was cooled to room temperature.

### Workup:

To the reaction mixture water and toluene were added. It was stirred for 10-15 min. and transferred into a separating funnel. Organic layer was collected. The product was extracted with 20 % aqueous HCl solution. It was basified with 40 % aqueous K₂CO₃ solution at 15-20 °C. The product was extracted with ethyl acetate. Both the extracts were combined and washed with brine solution. Organic layer was collected and dried over anhydrous sodium sulfate and solvent was distilled out at reduced pressure.

(+)-(1S,3'R)-quinuclidin-3'-yl-1-phenyl-1,2,3,4-tetrahydro-isoquinoline-2-carboxylate (Solifenacin) (6.6 g , 51 % yield) was obtained.
% Chemical purity 96.87 %
% Chiral purity by HPLC - 98.83 %.

### Example 2

### Preparation of (+)-(1S,3'R)-quinuclidin-3'-yl 1-phenyl-1,2,3,4-tetrahydroisoquinoline-2-carboxylate (Solifenacin)

Sodium salt of (R)-3-quinuclidinol (8.6 g), toluene (70 mL) and DMF (11 mL) were added in a dry three neck round bottom flask under N₂ atmosphere. The mixture was stirred for 5-10 min. To this mixture sodium methoxide (1.8 g) was added. The mixture was stirred at 25-30 °C for 15-30 min. The reaction mixture was heated to reflux temperature and stirred for 1 h. During the process traces of water and methanol were removed azeotropically by using Dean-Stark apparatus. It was cooled to 70-75 °C. (S)-Ethyl 1-phenyl-1,2,3,4-tetrahydroisoquinoline-2-caboxylate (8.6 g) dissolved in dry toluene was added to the mixture. It was again heated to reflux temp. and stirred for 8 h while distilling off solvent to remove ethanol at intervals with addition of fresh quantity of dry solvent. It was cooled to room temperature. After suitable work-up as described in Example 1, (+)-(1S,3'R)-quinuclidin-3'-yl 1-phenyl-1,2,3,4-tetrahydro-isoquinoline-2-carboxylate (Solifenacin) (5.3 g , 50 % yield) was obtained.
% Chemical purity 97.67 %, % Chiral purity by HPLC - 99.15 %.

### Example 3

### Preparation of (+)-(1S,3'R)-quinuclidin-3'-yl 1-phenyl-1,2,3,4-tetrahydroisoquinoline-2-carboxylate (Solifenacin)

To a cooled solution of freshly prepared sodium methoxide (4.2 g), (R)-3-quinuclidinol (10 g) was added under N₂ atmosphere. It was stirred at 5-30 °C for 30 min Distilled out the solvent at reduced pressure. To the semi-solid mass dry toluene was added and reaction mixture was heated to reflux temperature and stirred for 1 to 2h. During the process, traces of water and methanol were removed azeotropically by using Dean-Stark apparatus, and mixture was cooled to 60-70 °C. (S)-Ethyl 1-phenyl-1,2,3,4-tetrahydroisoquinoline-2-caboxylate (20 g) dissolved in dry toluene (160 mL) and dry DMF (25 mL) were added. It was again heated to reflux temperature and stirred for 10 to 12 h while distilling off toluene to remove ethanol after every 15 min. intervals with addition of fresh quantity of dry toluene. It was cooled to room temperature. After suitable work-up similar to that described in Example 1, (+)-(1S,3'R)-quinuclidin-3'-yl 1-phenyl-1,2,3,4-tetrahydro-isoquinoline-2-carboxylate (Solifenacin) (18.2 g , 72 % yield) was obtained.
% Chemical purity 97.37 %, % Chiral purity by HPLC - 99 %.
SOR (1% in EtOH at 25 °C) : 123.2 °

### Example 4

### Preparation of (+)-(1S,3'R)-quinuclidin-3'-yl 1-phenyl-1,2,3,4-tetrahydroisoquinoline-2-carboxylate (Solifenacin)

To a cooled solution of freshly prepared sodium methoxide (4.2 g), (R)-3-quinuclidinol (10 g) was added under N₂ atmosphere. It was stirred at 5-30 °C for 30 min Distilled out the solvent at reduced pressure. To the semi-solid mass dry toluene was added. Reaction mixture was heated to reflux temperature and stirred for 1 to 2 hrs. During the process, traces of water and methanol were removed azeotropically by using Dean-Stark apparatus. It was cooled to 60-70 °C. (S)- Ethyl 1-phenyl-1,2,3,4-tetrahydroisoquinoline-2-caboxylate (20 g) dissolved in dry toluene(160 mL) and dry DMF (25 mL) were added and was again heated to reflux temp. and stirred for 10 to12 hrs while distilling off toluene to remove ethanol after every 15 min. intervals with addition of fresh dry toluene. It was cooled to room temperature. After suitable work-up similar to that described in Example 1, (+)-(1S,3'R)-quinuclidin-3'-yl 1-phenyl-1,2,3,4-tetrahydro-isoquinoline-2-carboxylate (Solifenacin) (21 g , 81.7 % yield) was obtained.
% Chemical purity 96.76 %, % Chiral purity by HPLC - 95.27 %.
SOR (1 % in EtOH at 25 °C): 118.5 °

### Example 5

### Preparation of (+)-(1S,3'R)-quinuclidin-3'-yl 1-phenyl-1,2,3,4-tetrahydroisoquinoline-2-carboxylate (Solifenacin)

To a cooled methanolic solution of sodium methoxide (2.1 g), (R)-3-quinuclidinol (5 g) was added under N₂ atmosphere. It was stirred at 5-30 °C for 30 min to 1 hrs and distilled out the solvent at reduced pressure to obtain semi solid mass. To the semi-solid mass dry toluene was added and reaction mixture was heated to reflux temp. and stirred for 1-3 h. During the process traces of water and methanol were removed azeotropically by using Dean-Stark apparatus. It was cooled to 60-70 °C. (S)-Ethyl 1-phenyl-1,2,3,4-tetrahydroisoquinoline-2-caboxylate (10 g) dissolved in dry toluene and dry DMF were added. It was again heated to reflux temperature and stirred for 12 to13 h while distilling off solvent to remove ethanol at intervals with addition of fresh quantity of dry solvent. It was cooled to room temperature. After suitable work-up similar to that described in Example 1, (+)-(1S,3'R)-quinuclidin-3'-yl-1-phenyl-1,2,3,4-tetrahydroisoquinoline-2-carboxylate (Solifenacin) (9.3 g, 72 % yield) was obtained.
% Chemical purity 95.79 %; % Chiral purity by HPLC - 96.0 %.
SOR (1% in EtOH at 25 °C) : 123.1 °

### Example 6

### Preparation of (+)-(1S,3'R)-quinuclidin-3'-yl 1-phenyl-1,2,3,4-tetrahydroisoquinoline-2-carboxylate (Solifenacin)

To a cooled solution of freshly prepared sodium ethoxide (0.9 g), (R)-3-quinuclidinol (5 g) was added under N₂ atmosphere. It was stirred at 5-30 °C for 30 min to 1hrs and distilled out the solvent at reduced pressure. To the semi-solid mass dry toluene was added. Reaction mixture was heated to reflux temperature and stirred for 1-3 h. During that traces of water and ethanol were removed azeotropically by using Dean-Stark apparatus. It was cooled to 60-70 °C. (S)- Ethyl 1-phenyl-1,2,3,4-tetrahydroisoquinoline-2-caboxylate (10 g) dissolved in dry toluene and dry DMF were added. It was again heated to reflux temperature and stirred for 9 to 10 hrs while distilling off solvent to remove ethanol at intervals with addition of fresh dry solvent. It was cooled to room temperature. After suitable work-up similar to that described in Example 1, (+)-(1S,3'R)-quinuclidin-3'-yl I-phenyl-1,2,3,4-tetrahydro-isoquinoline-2-carboxylate (Solifenacin) (6.3 g , 50 % yield) was obtained. % Chemical purity 95.68 %, % Chiral purity by HPLC - 97.19 %.

### Example 7

### Preparation of (+)-(1S,3'R)-quinuclidin-3'-yl 1-phenyl-1,2,3,4-tetrahydroisoquinoline-2-carboxylate (Solifenacin)

To a cooled solution of freshly prepared sodium methoxide (0.45 g), (R)-3-quinuclidinol (2.5 g) was added under N₂ atmosphere. It was stirred at 5-30 °C for 30 min to 1 hrs and distilled out the solvent at reduced pressure. To the semi-solid mass dry toluene was added. Reaction mixture was heated to reflux temp. and stirred for 1-3 h. During the process, traces of water and methanol were removed azeotropically by using Dean-Stark apparatus. It was cooled to 60-70 °C. (S)-Ethyl 1-phenyl-1,2,3,4-tetrahydroisoquinoline-2-caboxylate (5 g) dissolved in dry toluene (40 mL) and dry N,N'-dimethyl acetamide (6.2 mL) were added. It was again heated to reflux temperature and stirred for 9 to 10 hrs while distilling off solvent to remove ethanol at intervals with addition of fresh dry solvent. It was cooled to room temperature. After suitable work-up as described in Example 1, (+)-(1S,3'R)-quinuclidin-3'-yl 1-phenyl-1,2,3,4-tetrahydroisoquinoline-2-carboxylate (Solifenacin) (4.2 g, 65 %yield) was obtained.
% Chemical purity 93.38 %, % Chiral purity by HPLC - 92.72 %.

### Example 8

### Preparation of (+)-(1S,3'R)-quinuclidin-3'-yl 1-phenyl-1,2,3,4-tetrahydroisoquinoline-2-carboxylate (Solifenacin)

To a cooled solution of freshly prepared sodium methoxide (2.1 g), (R)-3-quinuclidinol (5 g) was added under N₂ atmosphere. It was stirred at 5-30 °C for 30 min to 1 hrs and distilled out the solvent at reduced pressure. To the semi-solid mass dry toluene was added. Reaction mixture was heated to reflux temperature and stirred for 1-3 h. During that traces of water and methanol were removed azeotropically by using Dean-Stark apparatus. It was cooled to 60-70 °C. (S)- Ethyl 1-phenyl-1,2,3,4-tetrahydroisoquinoline-2-caboxylate (10 g) dissolved in dry toluene (80 mL) and dry MIBK (12.5 mL) were added. It was again heated to reflux temp. and stirred for 9 to 10 hrs while distilling off solvent to remove ethanol at intervals with addition of fresh dry solvent. It was cooled to room temperature. After suitable work-up as described in Example 1, (+)-(1S,3'R)-quinuclidin-3'-yl 1-phenyl-1,2,3,4-tetrahydro-isoquinoline-2-carboxylate (Solifenacin) (2.0g, 16% yield) was obtained. % Chemical purity- 66.7 %)

### Example 9

### Preparation of (+)-(1S,3'R)-quinuclidin-3'-yl 1-phenyl-1,2,3,4-tetrahydroisoquinoline-2-carboxylate (Solifenacin)

To a cooled solution of freshly prepared sodium methoxide (2.1 g), (R)-3-quinuclidinol (2.5 g) was added under N₂ atmosphere. It was stirred at 5-30 °C for 15-30 min. Distilled out the solvent at reduced pressure. To the semi-solid mass dry toluene was added and reaction mixture was heated to reflux temp. and stirred for 1-3 h. During the process, traces of water and methanol were removed azeotropically by using Dean-Stark apparatus. It was cooled to 50-60 °C. (S)- Ethyl 1-phenyl-1,2,3,4-tetrahydroisoquinoline-2-caboxylate (5 g) dissolved in dry toluene (20 mL) and dry THF (6.0 mL) were added. It was again heated to reflux temperature and stirred for 9 to 10 hrs while distilling off solvent to remove ethanol at intervals with addition of fresh mixture of toluene-THF. The mixture was cooled to room temperature. After suitable work-up similar to that described in Example 1, (+)-(1S,3'R)-quinuclidin-3'-yl 1-phenyl-1,2,3,4-tetrahydro-isoquinoline-2-carboxylate (Solifenacin) (3 g , 46.5 % yield) was obtained. % Chemical purity- 92.1 %)

### Example 10

### Preparation of (+)-(1S,3'R)-quinuclidin-3'-yl 1-phenyl-1,2,3,4-tetrahydroisoquinoline-2-carboxylate (Solifenacin)

In a dry three neck round bottom flask, (R)-3-quinuclidinol (5.0 g) toluene (80 mL) and DMF (12.5 mL) were added under N₂ atmosphere. It was stirred for 5-10 min. To this mixture sodium methoxide (3.2 g) was added. It was stirred at 25-30 °C for 15-30 min. Reaction mixture was heated to reflux temp. and stirred for 1 to 2 hrs. During the process, traces of water and methanol were removed azeotropically by using Dean-Stark apparatus. It was cooled to 70-75 °C. (S)- Ethyl 1-phenyl-1,2,3,4-tetrahydroisoquinoline-2-caboxylate (10 g) dissolved in dry toluene was added. It was again heated to reflux temperature and stirred for 8 to 9 hrs while distilling off solvent to remove ethanol at intervals with addition of fresh dry solvent. It was cooled to room temperature. After suitable work-up similar as described in Example 1; (+)-(15,3'R)-quinuclidin-3'-yl 1-phenyl-1,2,3,4-tetrahydro-isoquinoline-2-carboxylate (Solifenacin)(5 g , 40 % yield) was obtained.
% Chemical purity 92.75 %, % Chiral purity by HPLC - 96.4 %.

### Example 11

### Preparation of (+)-(1S,3'R)-quinuclidin-3'-yl 1-phenyl-1,2,3,4-tetrahydroisoquinoline-2-carboxylate (Solifenacin)

To a cooled solution of (R)-3-quinuclidinol (10 g) in dry toluene (80 mL) and dry DMF(12.5 mL), NaH (1.7 g) was added under N₂ atmosphere. It was stirred at 5-30 °C for 30 min. (S)-Ethyl 1-phenyl-1,2,3,4-tetrahydroisoquinoline-2-caboxylate (10 g) was dissolved in dry toluene and reaction mixture was heated to reflux temperature and stirred for 9 to 10 hrs while distilling off solvent to remove ethanol at intervals with addition of fresh dry solvent. The mixture was cooled to room temperature. After suitable work-up as described in Example 1, (+)-(1S,3'R)-quinuclidin-3'-yl-1-phenyl-1,2,3,4-tetrahydro-isoquinoline-2-carboxylate (Solifenacin) (8.2 g , 64 % yield) was obtained.
% Chemical purity 97.59 %, % Chiral purity by HPLC-97 %.

### Example 12

### Preparation of mixture of (1S,3'R),(1R,3'R) and (1S,3'S), (1R,3'S)-quinuclidin-3'-yl 1-phenyl-1,2,3,4-tetrahydro-isoquinoline-2-carboxylate

A solution of (3R/S)-quinuclidinol (0.5 g) in 20 mL toluene was heated to reflux temperature and stirred for 1-3 h. During that traces of water was removed azeotropically by using Dean-Stark apparatus. It was cooled to 40- 50 °C and 55 % NaH (0.2 g) in dry DMF was added. Reaction mix. was stirred at 100 °C. After 1 to 2 hrs it was cooled to 50 °C and (1R/S)- Ethyl 1-phenyl-1,2,3,4-tetrahydroisoquinoline-2-caboxylate (1 g) dissolved in dry toluene was added. It was again heated to reflux temperature and stirred for 5-25 h while distilling off solvent to remove ethanol at intervals with addition of fresh dry solvent. It was cooled to room temperature. To the reaction mixture brine was added. It was stirred for 10-15 min. and transferred into a separating funnel. It was extracted with ethyl acetate. Organic layer was collected. The product was extracted with 20 % aqueous HCl solution. Aqueous layer was collected and basified with 1M Aqueous NaOH solution at 15-20 °C. The product was extracted with ethyl acetate. All the extracts were combined and dried over anhydrous Sodium sulfate. Solvent was distilled out at reduced pressure. Diastereomeric mixture of quinuclidin-3'-yl 1-phenyl-1,2,3,4-tetrahydro-isoquinoline-2-carboxylate (0.6 g , 50 % yield) was obtained.
% Chemical purity 92.32 %

### Example 13

### Preparation of mixture of (1R,3'R) and (1R,3'S)-quinuclidin-3'-yl 1-phenyl-1,2,3,4-tetrahydro-isoquinoline-2-carboxylate

A solution of (3R/S)-quinuclidinol (2.3 g) in 20 mL toluene was heated to reflux temp. and stirred for 1-3 h. During the process, traces of water was removed azeotropically by using Dean-Stark apparatus. It was cooled to 40- 50 °C and 55 % NaH (0.2 g) in dry DMF was added. Reaction mix. was stirred at 100 °C. After 1h it was cooled to 50 °C and (1R/S)-Ethyl 1-phenyl-1,2,3,4-tetrahydroisoquinoline-2-caboxylate (1.54 g) dissolved in dry toluene was added. It was again heated to reflux temp. and stirred for 5-25 h while distilling off solvent to remove ethanol at intervals with addition of fresh dry solvent. It was cooled to room temperature. After suitable work-up similar to that described in Example 12, diastereomeric mixture of Quinuclidin-3'-yl 1-phenyl-1,2,3,4-tetrahydro-isoquinoline-2-carboxylate (1.1 g, 55 % yield) was obtained.
% Chemical purity 95.39 %

### Example 14

### Preparation of (-)-(1R,3'R)-quinuclidin-3'-yl 1-phenyl-1,2,3,4-tetrahydroisoquinoline-2-carboxylate

To a cooled solution of freshly prepared sodium methoxide (0.56 g), (R)-3-quinuclidinol.HCl (3.1 g) was added under N₂ atmosphere. It was stirred at 5-30 °C for 30 min Distilled out the solvent at reduced pressure. To the semi-solid mass dry toluene (30 mL) was added. Reaction mixture was heated to reflux temp. and stirred for 1-3 h. During the process, traces of water was removed azeotropically by using Dean-Stark apparatus. It was cooled to 60- 70 °C. (1R)- Ethyl 1-phenyl-1,2,3,4-tetrahydroisoquinoline-2-caboxylate (2 g) dissolved in dry toluene and dry DMF were added. It was again heated to reflux temp. and stirred for 5-25 h while distilling off solvent to remove ethanol at intervals with addition of fresh dry solvent. It was cooled to room temperature. After suitable work-up similar to that described in Example 12, (-)-(1R,3'R)-quinuclidin-3'-yl-1-phenyl-1,2,3,4-tetrahydro-isoquinoline-2-carboxylate (2.5 g , 62 % yield) was obtained.
% Chemical purity 93.43 %, % Chiral purity by HPLC - 97 %.

### Example 15

### Preparation of (+)-(1S,3'R)-quinuclidin-3'-yl 1-phenyl-1,2,3,4-tetrahydroisoquinoline-2-carboxylate (Solifenacin) from (S) 1-Phenyl-1,2,3,4-tetrahydroisoquinoline

In a three neck round bottom flask, toluene (80 mL), (S) 1-phenyl-1,2,3,4-tetrahydroisoquinoline (16.2 g) and potassium carbonate (11.2,g) dissolved in water were added. Reaction mixture was cooled at 0-5 °C with stirring. To the solution ethyl chloroformate (7.8 mL) was added drop by drop over a period of 15 min. at 5-10 °C. After the addition was completed, the mixture was warmed up to 25-30 °C and stirred for 3 to 4 hrs, after that it was diluted with water and transferred into a separating funnel. Organic layer was collected and washed with water and brine solution, and dried over anhydrous sodium sulfate. This solution containing (S)-Ethyl 1-phenyl-1,2,3,4-tetrahydroisoquinoline-2-caboxylate was used in following reaction.

In a dry three neck round bottom flask, (R)-3-quinuclidinol (10.0 g) toluene (160 mL) and DMF (25.0 mL) were added under N₂ atmosphere. It was stirred for 5-10 min. To this mixture sodium methoxide (4.3 g) was added. It was stirred at 25-30 °C for 15-30 min. Reaction mixture was heated to reflux temperature and stirred for 1 to 2 hrs. During the process, traces of water and methanol were removed azeotropically by using Dean-Stark apparatus. It was cooled to 50-60 °C. The solution of (S)-Ethyl 1-phenyl-1,2,3,4-tetrahydroisoquinoline-2-caboxylate in dry toluene was added (prepared above). It was again heated to reflux temp. and stirred for 9 to 10 hrs while distilling off solvent to remove ethanol at intervals with. addition of fresh dry solvent. It was cooled to room temperature. After suitable work-up as described in Example 1, (+)-(1S,3'R)-quinuclidin-3'-yl 1-phenyl-1,2,3,4-tetrahydro-isoquinoline-2-carboxylate (Solifenacin) (19.5 g , 69.5 % yield of two steps) was obtained.
% Chemical purity 97.2 %, % Chiral purity by HPLC - 96.8 %.

### Example 16

### Crystallization of (+)-(1S,3'R)-quinuclidin-3'-yl 1-phenyl-1,2,3,4-tetrahydroisoquinoline-2-carboxylate (Solifenacin base)

In a dry, 25 mL round bottom flask acetonitrile (1.5 mL), (+)-(1S,3'R)-quinuclidin-3'-yl 1-phenyl-1,2,3,4-tetrahydro-isoquinoline-2-carboxylate (3.0 g, % purity- 96.9 and % chiral purity-98.8 %) were taken. It was warmed. To the clear solution diisopropyl ether (6.0 mL) was added and stirred at 0-5 °C for 24 hrs. Solid was precipitated, filtered and washed with cold diisopropyl ether and dried to obtain the solid base (Wt.- 0.350 g, % purity- 99.0% and % chiral purity-99.8 %). m.p: 89 ± 2 °C
SOR (1 % in EtOH at 25 °C): 134.2 °

### Example 17

### Crystallization of (+)-(1S,3'R)-quinuclidin-3'-yl 1-phenyl-1,2,3,4-tetrahydroisoquinoline-2-carboxylate (Solifenacin base)

To a dry, 25 mL round bottom flask acetonitrile (1.5 mL), (+)-(15,3'R)-quinuclidin-3'-yl 1-phenyl-1,2,3,4-tetrahydro-isoquinoline-2-carboxylate (3.0 g, % purity- 93.6 and % chiral purity-97.4 % ) were taken. It was warmed. To the clear solution diisopropyl ether (6.0 mL) was added. and stirred at 0-5 °C for 24 h. solid did not precipitated. It was further cooled to -40 to -30 °C. Solid was precipitated. It was filtered at -30°C and washed with a cold (-40 to -30 °C) diisopropyl ether and dried to obtain the solid base (Wt.- 0.850 g, % purity- 98.5 and % chiral purity-99.5 %). m.p: 89 ±2°C.

### Example 18

### Crystallization of (+)-(1S,3'R)-quinuclidin-3'-yl 1-phenyl-1,2,3,4-tetrahydroisoquinoline-2-carboxylate (Solifenacin base)

In a dry, 50 mL round bottom flask DIPE (25.0 mL) and (+)-(1S,3'R)-quinuclidin-3'-yl 1-phenyl-1,2,3,4-tetrahydro-isoquinoline-2-carboxylate (5.0 g, % purity- 95.9 and % chiral purity-97.9 % ) were taken. It was warmed to dissolve the material. It was filtered while hot through hyflo bed to obtain clear solution. The clear filtrate was transferred into a single neck flask and it was concentrated upto approximately 20 mL volume. After that it was cooled to 0-5 °C with stirring. After 1 h solid got precipitated To the thick slurry another batch of DIPE (5 mL) was added and stirred for 30-60 min. at 0-5 °C. It was filtered and washed with a cold diisopropyl ether (10 mL) and dried to obtain the solid base (Wt.- 2.8 g, % purity- 98.6 and % chiral purity-99.9 %). m.p: 89 ± 2 °C

The crystalline form of Solifenacin base was characterized by PXRD peaks at about 7.52, 13.0, 14.68, 15.15, 16.02, 18.16, 19.6, 20.36, 20.76, 22.68, 24.45,26.62, 26.92 °± 2° 2θ.

### Example 19

### Preparation of salt of (+)-(15,3'R)-quinuclidin-3'-yl 1-phenyl-1,2,3,4-tetrahydroisoquinoline-2-carboxylate (Solifenacin base) with (-) Di-p-toluoyl-L-tartaric acid

To an ethanolic solution of (+)-(15,3'R)-quinuclidin-3'-yll-phenyl-1,2,3,4-_ tetrahydro-isoquinoline-2-carboxylate (5.0 g, % chiral purity-98.1) , (-) Di-p-toluoyl-L-tartaric acid (5.33 g) was added. It was warmed and stirred for 60-120 min. Distilled out the solvent at reduced pressure. To the semi-solid mass dry toluene was added and distilled out. Solid salt was obtained (Wt.- 10.3 g)

The salt (2.0 g) was stirred with isopropyl acetate with heating for 30-60 min. It was cooled to room temperature. It was filtered and washed with isopropyl acetate and dried to obtain the solid salt (Wt.-1.46 g, % purity-97 %, % chiral purity-99.7 %). M. p.: 145-147 °C.

### Example 20

### Preparation of salt of (+)-(1S,3'R)-quinuclidin-3'-yl 1-phenyl-1,2,3,4-tetrahydroisoquinoline-2-carboxylate (Solifenacin base) with (-) Di-p-toluoyl-L-tartaric acid

To an ethanolic solution of (+)-(1S,3'R)-quinuclidin-3'-yl 1-phenyl-1,2,3,4-tetrahydro-isoquinoline-2-carboxylate (5.0 g, % chiral purity-98.1) , (-) Di-p-toluoyl-L-tartaric acid (5.33 g) was added. It was warmed and stirred for 60-120 min. Distilled out the solvent at reduced pressure. To the semi-solid mass dry toluene was added and distilled out. Solid salt was obtained (Wt.- 10.3 g)

The salt (1.0 g) was dissolved in acetonitrile at 70-82 °C. It was cooled to 0-5 °C and stirred until the solid was precipitated. To the suspension, acetonitrile was added and stirred at 25-30 °C. It was filtered and washed with cold acetonitrile and dried to obtain the solid salt (Wt.- 0.2 g, % purity-97 %, % chiral purity-99.6 %).

### Example 21

### Preparation of (+)-(1S,3'R)-quinuclidin-3'-yl 1-phenyl-1,2,3,4-tetrahydroisoquinoline-2-carboxylate (Solifenacin base) via (-) Di-p-toluoyl-L-tartaric acid salt

To an ethanolic solution of (+)-(1S,3'R)-quinuclidin-3'-yl 1-phenyl-1,2,3,4-tetrahydro-isoquinoline-2-carboxylate (5.0 g, % chiral purity-99.5) , (-) Di-p-toluoyl-L-tartaric acid (5.33 g) was added. It was warmed and stirred for 60-120 min. Distilled out the solvent at reduced pressure. To the semi-solid mass dry toluene was added and distilled out. Solid salt was obtained (Wt.-10.3 g)

The salt (7.2 g) was stirred with acetonitrile at 25-30 °C for 30-60 min.. It was filtered and washed with cold acetonitrile and dried to obtain the solid salt (Wt.- 5.0 g)

The Di-p-toluoyl-L-tartaric acid salt (5.0 g) was added to ethyl acetate and cooled to 0-5 °C. To the mixture sodium bicarbonate (0.6 g) dissolved in water was added at 0-5 °C. It was stirred for 30-60min. at 25-30 °C. Organic layer was separated. Aqueous layer was again extracted with ethyl acetate. Both the extracts were combined and washed with water and brine solution. Organic layer was collected and dried over anhydrous Sodium sulfate. It was concentrated at reduced pressure to obtain Solifenacin base (Wt.- 2.4 g, % purity-97 %, chiral purity-100 %).

### Example 22

### Preparation of (+)-(15,3'R)-quinuclidin-3'-yl 1-phenyl-1,2,3,4-tetrahydroisoquinoline-2-carboxylate (Solifenacin base) via (-) Di-p-toluoyl-L-tartaric acid salt

To an ethanolic solution of (+)-(1S,3'R)-quinuclidin-3'-yl 1-phenyl-1,2,3,4-tetrahydro-isoquinoline-2-carboxylate (5.0 g, % chiral purity-96.0 %) , (-) Di-p-toluoyl-L-tartaric acid (5.33 g) was added. It was warmed and stirred for 60-120 min. Distilled out the solvent at reduced pressure. To the semi-solid mass dry toluene was added and distilled out. Solid salt was obtained (Wt.-10.3 g)

The salt (5.0 g) was stirred with acetonitrile at 25-30 °C for 30-60 min.. It was filtered and washed with cold acetonitrile and dried to obtain the solid salt (Wt.-4.0 g)

Di-paratoluoyl-L-tartaric acid salt of Solifenacin was characterized by a PXRD pattern with peaks at about 5.67, 11:49, 12.82, 13.49, 13.97, 15.01, 15.68, 16.00, 17.80, 18.28, 19.10, 20.38, 22.36, 23.08, 23.94, 24.50, 24.94 ° ± 2 °2θ, (Fig-10).

The Di-p-toluoyl-L-tartaric acid salt (3.75 g) was added to ethyl acetate and cooled to 0-5 °C. To the mixture sodium bicarbonate (0.8 g) dissolved in water was added at 0-5 °C. It was stirred for 30-60min. at 25-30 °C. Organic layer was separated. It was again stirred with a solution of sodium bicarbonate (0.8 g) dissolved in water for 30-60 min. at 25-30 °C. Organic layer was separated. It was washed with water and brine solution. Organic layer was collected and dried over anhydrous. Sodium sulfate. It was concentrated at reduced pressure to obtain Solifenacin base.
(Wt.-1.75g, % Purity-98.95, % chiral purity-100 %).

### Example 23

### Preparation of (+)-(1S,3'R)-quinuclidin-3'-yl 1-phenyl-1,2,3,4-tetrahydroisoquinoline-2-carboxylate gentisic acid salt

In a dry, 25 mL round bottom flask ethanol (45 mL), (+)-(1S,3'R)-quinuclidin-3'-yl 1-phenyl-1,2,3,4-tetrahydro-isoquinoline-2-carboxylate (5.0 g, % chiral purity- 97.5 %) were taken. To the clear solution gentisic acid (2.13 g) was added. It was warmed and stirred for 60-120 min. The solvent was distilled out at reduced pressure to obtain the semi-solid mass. Toluene was added and distilled out. The above steps were repeated till solid salt was obtained (Wt.- 6.8 g).

The salt (6.8 g) was dissolved in ethyl acetate at reflux temperature. It was cooled to 25-30 °C. It was stirred for 2 to 3 hrs. Solid salt was precipitated. It was filtered and washed with cold ethyl acetate and dried to obtain the solid salt (Wt.- 5.5 g, % chiral purity-100 %).
M. p.: 172-174 °C, SOR (1 % in MeOH at 25 °C) : 65.7 °

### Example 24

### Preparation of (+)-(1S,3'R)-quinuclidin-3'-yl 1-phenyl-1,2,3,4-tetrahydroisoquinoline-2-carboxylate gentisate salt

In a dry, 25 mL round bottom flask ethanol (160 mL), (+)-(15,3'R)-quinuclidin-3'-yl. 1-phenyl-1,2,3,4-tetrahydro-isoquinoline-2-carboxylate (17.5 g, % chiral purity-97.5 % ) were taken. To the clear solution gentisic acid (7.45 g) was added. It was warmed and stirred for 60-120 min. The solvent was distilled out at reduced pressure to obtain the semi-solid mass. Toluene was added and distilled out. The above steps were repeated till solid salt was obtained (Wt.- 24.7 g)

The salt (24.7 g) was dissolved in ethyl acetate at reflux temperature. It was cooled to 25-30 °C. It was stirred for 2 to 3 hrs. Solid salt was precipitated. It was filtered and washed with cold ethyl acetate and dried to obtain the solid salt (Wt.- 19.5 g, % chiral purity-100 %). M. p.: 170-172 °C, SOR (1 % in MeOH at 25 °C) : 68.4 °

The crystalline form of Solifenacin gentisate was characterized by a PXRD. Sample was examined for crystallinity by powder X-ray diffraction and it was found that degree of crystallinity is 87.4 %. Sample was tested to determine the level of residual solvent, ethanol was found to be 0.33 % by weight and dimethyl formamide was 0.040 % by weight.

### Example 25

### Preparation of (+)-(15,3'R)-quinuclidin-3'-yl 1-phenyl-1,2,3,4-tetrahydroisoquinoline-2-carboxylate gentisate salt

To a dry, 500 mL round bottom flask ethanol (154 mL), (+)-(1S,3'R)-quinuclidin-3'-yl 1-phenyl-1,2,3,4-tetrahydro-isoquinoline-2-carboxylate (15.4 g, % chiral purity-99.8 % ) were taken. To the clear solution gentisic acid (6.5 g) was added. It was warmed and stirred for 60-120 min. The solvent was distilled out at reduced pressure to obtain the semi-solid mass. Toluene was added and distilled out. The above steps were repeated till solid salt was obtained (Wt.- 21.0 g)

The salt (21.0 g) was dissolved in ethyl acetate at reflux temperature. It was cooled to 0-5 °C. It was stirred for 1 to 2 hrs. Solid salt was precipitated. It was filtered and washed with cold ethyl acetate and dried to obtain the solid salt (Wt.- 17.0 g, % chiral purity-100 %). M. p.: 171-174 °C, SOR (1 % in MeOH at 25 °C) : 71.0°,

The crystalline form of Solifenacin gentisate was characterized by a PXRD pattern with peaks at about 5.56, 7.06, 7.70, 10.15, 14.63, 15.54, 17.63, 19.4, 19.7, 20.08, 20.68,
21.58, 25.48 ° ±.0.2 ° (2θ), (Fig-7). Sample was examined for crystallinity by powder X-ray diffraction and it was found that degree of crystallinity is 98.3 %.

### Example 26

### Preparation of (+)-(1S,3'R)-quinuclidin-3'-yl 1-phenyl-1,2,3,4-tetrahydroisoquinoline-2-carboxylate gentisate salt

In a dry, 25 mL round bottom flask ethanol (30 mL), a diastereomeric mixture of (1S,3'R) and (1R,3'R)-quinuclidin-3'-yl 1-phenyl-1,2,3,4-tetrahydro-isoquinoline-2-carboxylate (3.0 g) were taken. To the clear solution gentisic acid (1.18 g) was added. It was warmed and stirred for 60 to 90 min. Distilled out the solvent at reduced pressure to obtained the semi-solid mass. Toluene was added and distilled out. The gentisate salt was dissolved in ethyl acetate at reflux temperature. It was cooled to 0-5 °C and stirred for 60 to 90 min. Seeding was added. It was kept at 0-5 °C overnight. To the solution di isopropyl ether was added. Semi-solid mass separated. kept it for 10 days at 0-5 °C.. Solvent was decanted and semi-solid mass stirred with ethyl acetate for 2 to 3 hrs at 0-5 °C. Solid separated. It was filtered and washed with cold ethylacetate. Solid gentisate salt was obtained (Wt.- 0.800 g, % Yield-19 %, % Purity by HPLC-99.6 %, % Chiral purity-100 %).
M. p.: 164-165 °C, SOR (1 % in MeOH at 25 °C) : 68.4 °

### Crystallization of (+)-(1S,3'R)-quinuclidin-3'-yl 1-phenyl-1,2,3,4-tetrahydro-isoquinoline-2-carboxylate gentisate salt

The gentisate salt of (+)-(1S,3'R)-quinuclidin-3'-yl 1-phenyl-1,2,3,4-tetrahydroisoquinoline-2-carboxylate was crystallized in following solvents. The results are summarized in the table 5 given below,

**Table 5:**

| **Example No.** | **Input** | **Solvent** | **Output** | **% Purity by HPLC** | **% yield** | **m.p.** |
|---|---|---|---|---|---|---|
| **27** | 0.5 g | **IPA-EtOAc** | 0.31 g | 99.6% | 63 % | 173-174 °C |
| **28** | 0.5 g | **IPA** | 0.1 g | 99.8 % | 20 % | 171-172 °C |
| **29** | 0.5 g | **Acetone EtOAc** | 0.13 g | 99.8 % | 27 % | 171-173 °C |
| **30** | 0.5 g | **CHCl₃- EtOAc** | 0.39 g | 99.8 % | 78 % | 172-173 °C |
| **31** | 0.5 g | **ACN DIPE** | 0.33 g | 99.8 % | 66% | 171-172 °C |
| **32** | 0.5 g | **DMF Water** | 0.21 g | 99.9 % | 42% | 172-173 °C |

### Example 33

### Preparation of (+)-(1S,3'R)-quinuclidin-3'-yl 1-phenyl-1,2,3,4-tetrahydroisoquinoline-2-carboxylate oxalate salt

In a dry 25 mL round bottom flask ethanol(5 mL), (+)-(1S,3'R)-quinuclidin-3'-yl 1-phenyl-1,2,3,4-tetrahydro-isoquinolinc-2-carboxylate (1.0 g, % chiral purity-97.5%) were taken. To the clear solution oxalic acid dihydrate (0.348 g) was added. It was warmed and stirred for 60-120 min. Distilled out the solvent at reduced pressure. To the semi-solid mass toluene was added and distilled out when solid salt was obtained which was crystallized from a mixture of ethanol-ethyl acetate to obtain the salt with % chiral purity-99.0 % (Wt.- 1.20 g, %). m.p. 169-171 °C.

### Example 34

### Preparation of new polymorph of (+)-(1S,3'R)-quinuclidin-3'-yl 1-phenyl-1,2,3,4-tetrahydro-isoquinoline-2-carboxylate oxalate salt

In a dry, 1L round bottom flask ethanol (190 mL) and (+)-(1S,3'R)-quinuclidin-3'-yl 1-phenyl-1,2,3,4-tetrahydro-isoquinoline-2-carboxylate (19.0 g, % chiral purity-97.5) were taken. To the clear solution oxalic acid dihydrate (6.28 g) was added. It was heated to 70 °C and stirred for 60 min. Distilled out the solvent at reduced pressure. To the semi-solid mass dry toluene was added and subsequently distilled out. Again dry toluene was added and distilled out to obtain the solid salt. (Wt.- 25.0 g)

The salt (25 g) was dissolved in a mixture ethyl acetate and ethanol at 70-75 °C. It was slowly cooled to 25-30 °C. It was further cooled to 0-2 °C and it was stirred for 30 to 60 min. It was filtered at 0-2 °C and washed with cold ethyl acetate. White crystalline product was obtained. (Wt.- 18.8 g, % chiral purity > 99.0 %). m.p. 169-171 °C.
SOR (1 % in water at 25 °C): 42.35 °

The Oxalate salt of Solifenacin (16 g) was further stirred with ethyl acetate for 15 min. at reflux temperature. It was cooled to 0-2 °C. It was filtered at 0-2 °C and washed with cold ethyl acetate. It was dried. White crystalline product. (Wt.- 15.0 g, % chiral purity > 99.0%) was obtained.

The crystalline form of Solifenacin oxalate was characterized by PXRD peaks at about 9.78, 12.23, 12.68, 13.42, 15.44, 18.18, 19.56, 20.58, 21.45 and 25.24 ° ± 0.2 ° (20). (Fig-4). Sample was examined for crystallinity by powder X-ray diffraction and it was found that degree of crystallinity is 87.7 %.
M. p.: 171-174 °C; SOR ( 1 % in water at 25 °C): 43.2 °

### Example 35

### Preparation of (+)-(1S,3'R)-quinuclidin-3'-yl 1-phenyl-1,2,3,4-tetrahydroisoquinoline-2-carboxylate Oxalate salt

In a dry, 50 mL round bottom flask ethanol (15 mL) and (+)-(15,3'R)-quinuclidin-3'-yl 1-phenyl-1,2,3,4-tetrahydro-isoquinoline-2-carboxylate (1.4 g, % chiral purity-100) were taken. To the clear solution oxalic acid dihydrate (0.5 g) was added. It was heated to 70 °C and stirred for 60 min. Distillled out the solvent at reduced pressure. To the semi-solid mass dry toluene was added and subsequently distilled out. Again dry toluene was added and distilled out to obtain the solid salt (Wt.-1.85 g)

The salt (1.85 g) was stirred in a mixture acetonitrile and diisopropyl ether for 15 min. at reflux temperature. It was gradually cooled to 25-30 °C. It was further cooled to 0-2 °C and it was stirred for 30 to 60 min. It was filtered at 0-2 °C and washed with cold diisopropylether . It was dried in an drying oven at 60 °C. White crystalline product was obtained. (Wt.- 1.5 g, % chiral purity - 100.0 %).
M. p.: 173-174 °C

### Example 36

### Preparation of (+)-(1S,3'R)-quinuclidin-3'-yl 1-phenyl-1,2,3,4-tetrahydroisoquinoline-2-carboxylate Oxalate salt

In a dry, 25 mL round bottom flask ethanol (20 mL) and (+)-(15,3'R)-quinuclidin-3'-yl 1-phenyl-1,2,3,4-tetrahydro-isoquinoline-2-carboxylate (2.0 g, % chiral purity-98.8) were taken. To the clear solution oxalic acid dihydrate (0.51 g) was added. It was heated to 70 °C and stirred for 60 min. Distilled out the solvent at reduced pressure. To the semi-solid mass dry toluene was added and subsequently distilled out. Again dry toluene was added and distilled out to obtain the solid salt (Wt.- 2.5 g)

The salt (2.5 g) was stirred in a mixture IPA and diisopropyl ether for 15 to 45 min. at reflux temperature. It was gradually cooled to 25-30 °C and it was stirred for 1 to 2 h. It was filtered and washed with cold diisopropyl ether. It was dried in an drying oven at 60 °C. White crystalline product was obtained. (Wt.- 2.0 g, % chiral purity - 99.9 %).
M. p.: 164-170 °C

### Crystallization of (+)-(1S,3'R)-guinuclidin-3'-yl 1-phenyl-1,2,3,4-tetrahydro-isoquinoline-2-carboxylate oxalate salt

The Oxalate salt of (+)-(1S,3'R)-quinuclidin-3'-yl 1-phenyl-1,2,3,4-tetrahydroisoquinoline-2-carboxylate was crystallized in following solvents. The results are summarized in a table given below:

**Table 6:**

| **Example No.** | **Input** | **Solvent** | **Output** | **% Purity by HPLC** | **% yield** | **m.p.** |
|---|---|---|---|---|---|---|
| **37** | 0.5 g | **CHCl₃-DIPE** | 0.285 g | 98.6% | 57 % | 169-171 °C |
| **38** | 0.5 g | **CHCl₃-Toluene** | 0.225 g | 99.0 % | 45 % | 172-173 °C |
| **39** | 0.5 g | **2-methoxy ethanol-EtOAc** | 0.325 g | 99.2 % | 65 % | 171-173 °C |
| **40** | 0.5 g | **CH₃CN** | 0.38 g | - | 76% | 172-173 °C |
| **41** | 0.5 g | **MeOH-EtOAc** | 0.12 g | - | 24 % | 172-173 °C |
| **42** | 0.5 g | **CHCl₃-IPA** | 0.22 g | - | 44 % | 170-171 °C |
| **43** | 0.5 g | **IPAc** | 0.38 g | - | 76% | 169-170 °C |

### Example 44

### Preparation of (+)-(1S,3'R)-quinuclidin-3'-yl 1-phenyl-1,2,3,4-tetrahydroisoquinoline-2-carboxylate hydrochloride salt

In a dry, 1.0 L round bottom flask ethanol (175 mL) and (+)-(1S,3'R)-quinuclidin-3'-yl 1-phenyl-1,2,3,4-tetrahydro-isoquinoline-2-carboxylate (20.5 g, % chiral purity-95.8) were taken. It was cooled to 5-10 °C. To the clear solution 24 % IPA-- HCl (8.7 mL) was added at 5-10 °C. It was warmed up to 25-30 °C and stirred for 1 h. Distilled out the solvent at reduced pressure. To the semi-solid mass toluene was added and distilled out. Solid salt was obtained (Wt.- 22.4 g %).

The salt (22.4 g) was dissolved in a mixture ethanol and ethyl acetate at 70-75 °C and stirred for 30 min. It was slowly cooled to 0-2 °C and was stirred for 90 to 120 min. The mixture was filtered at 0-2 °C and washed with cold ethyl acetate. It was dried at 65 °C in a drying oven. White crystalline product was obtained. (Wt.- 13.0 g, % chiral purity- 100.0 %), m.p. 262-266 °C.
SOR ( 1 % in ethanol at 25 °C): 99.39 °

The crystalline form of Solifenacin hydrochloride was characterized by PXRD peaks at about 9.61, 13.18, 14.02, 14.39, 15.58, 15.89, 17.0, 18.94, 19.18, 19.78, 20.98, 21.61 and 26.12 ° ±.0.2° ° (2θ). (Fig-1).

Sample was examined for crystallinity by powder X-ray diffraction and it was found that degree of crystallinity is 96.2 %.

### Crystallization of (+)-(1S,3'R)-quinuclidin-3'-yl 1-phenyl-1,2,3,4-tetrahydro-isoquinoline-2-carboxylate hydrochloride salt

The hydrochloride salt of (+)-(1S,3'R)-quinuclidin-3'-yl 1-phenyl-1,2,3,4-tetrahydro-isoquinoline-2-carboxylate was crystallized in the following solvents. The results are summarized in the table given below,

**Table 7:**

| **Example No.** | **Input** | **Solvent** | **Output** | **% Purity by HPLC** | **% yield** | **m.p.** |
|---|---|---|---|---|---|---|
| **45** | 0.2 g | **CHCl₃-DIPE** | 0.12 g | 97.8 % | 60% | 260-263 °C |
| **46** | 0.2 g | **IPA** | 0.130 g | - | 65 % | 260-263 °C |
| **47** | 0.2 g | **IPA-DMF** | 0.110 g | - | 55 % | 260-263 °C |

### Example 48

### Preparation of (+)-(1S,3'R)-quinuclidin-3'-yl 1-phenyl-1,2,3,4-tetrahydroisoquinoline-2-carboxylate succinate salt

The Solifenacin hydrochloride salt (5 g) was added to ethyl acetate (50 mL) and cooled to 0-5 °C. To the mixture sodium bicarbonate (2 g) dissolved in water (20 mL) was added at 0-5 °C. It was stirred for 30-60 min. at 25-30 °C. Organic layer was separated. It was washed with water and brine solution. Organic layer was collected and dried over anhydrous sodium sulfate. It was concentrated at reduced pressure to obtain Solifenacin base (Wt.- 4.5 g, % Purity-99.2, % Chiral purity-100 %).

The Solifenacin base (4.3 g) was dissolved in ethanol (43 mL). To the solution succinic acid (1.4 g) was added. It was heated to 70-75 °C. and stirred for 30 to 60 min. It was cooled to 25-30 °C. Distilled out the solvent at reduced pressure on Buchi rotavapour. To the semi-solid mass dry toluene was added and subsequently distilled out. Again dry toluene was added and distilled out to obtain the solid salt.

The salt was stirred in a mixture ethanol (9 mL) and ethyl acetate (45 mL) for 30 to 60 min. at 75-80 °C. It was gradually cooled to 25-30 °C and was stirred for 1 to 2 hrs. It was filtered and washed with cold ethyl acetate. It was dried in a drying oven at 60 °C. White crystalline product was obtained. (Wt.- 3.3 g, % chemical purity-99.66%, chiral purity - 100 %). m.p. 149-152 °C, SOR (1 % in ethanol at 25 °C): 87.2 ° .

### Preparation of Solifenacin succinate from Solifenacin oxalate:

Similarly the Solifenacin oxalate (5 g) was converted to Solifenacin succinate salt following similar procedure as above.

The Solifenacin base obtained from Solifenacin oxalate had % purity-99.01 % and % Chiral purity - 99.8 %

The Solifenacin succinate obtained had the following characteristics:
Wt.- 3.2 g, % chemical purity-99.3 %, chiral purity - 100 %;. m.p. 147-149 °C,
SOR (1% in ethanol at 25 °C): 91.6 °

Similarly the other salts of Solifenacin were converted to Solifenacin base with high chemical and chiral purity and then subsequently to Solifenacin succinate by processes similar to those described above.

### Example 49

### Preparation of sodium salt of (R)-3-quinuclidinol

To a cooled solution of freshly prepared sodium methoxide (2.1 g), (R)-3-quinuclidinol (5 g) was added under N₂ atmosphere. It was stirred at 5-30 °C for 30 to 60 min. Distilled out the solvent at reduced pressure. To the semi-solid mass dry toluene was added and subsequently distilled out. Again dry toluene was added and distilled out to obtain the solid salt. The solid salt was stirred with diisopropyl ether at 25-30 oC for 20 -30 min. It was filtered and washed with diisopropyl ether. White solid powder was obtained (Wt. - 5.8 g).

### Example 50

### Preparation of (S)- Ethyl 1-phenyl-1,2,3,4-tetrahydroisoquinoline-2-caboxylate

To a dry, 25 mL round bottom flask was charged (S)-1-phenyl-1,2,3,4-tetrahydro-isoquinoline (1 g) and acetone (6 mL) at room temperature (RT). It was cooled to 5 to 10 °C and ethyl chloroformate (0.52 g) was added slowly into the reaction mixture. It was heated to reflux temperature and stirred till completion of the reaction. Distilled out the solvent at reduced pressure. To the residue dil. Aq. HCl and dichloromethane were added. It was transferred into a separating funnel. Organic layer was collected. It was washed with saturated. Sodium bicarbonate solution. Organic layer was collected and dried over anhydrous. Sodium sulfate. It was concentrated under reduced pressure to obtain Ethyl (S)-1-phenyl-1,2,3,4-tetrahydro-isoquinoline-2-carboxylate. (1.2 g, % Yield-89.0 %), % Purity by HPLC- 97.7 % and % Chiral purity-99.1 %.

### Example 51

### Preparation of (+)-(1S,3'R)-quinuclidin-3'-yl 1-phenyl-1,2,3,4-tetrahydroisoquinoline-2-carboxylate gentisic acid salt

In a dry 25 mL round bottom flask ethanol (45 mL), (+)-(1S,3'R)-quinuclidin-3'-yl 1-phenyl-1,2,3,4-tetrahydro-isoquinoline-2-carboxylate (5.0 g, % chiral purity ≥ 97.5 % ) were taken. To the clear solution gentisic acid (2.13 g) was added. It was warmed and stirred for 60-120 min. The solvent was distilled out at reduced pressure to obtain the semi-solid mass.

Toluene was added and distilled out. The above steps were repeated till solid salt was obtained (Wt.- 6.8 g)

Similarly Solifenacin acid salt with following acids were prepared. Some of the salts of Solifenacin were obtained as liquid and some of the salts were obtained as solid. Results are summarized in the table as below:

**Table 8:**

| **Sr. No.** | **Acid** | **Nature** | **Remarks/M.P.** |
|---|---|---|---|
| **1** | α-Ketoglutaric acid | Solid | - |
| **2** | Glutaric acid | Solid | - |
| **3** | Pivalic acid | Thick liquid | - |
| **4** | Adipic acid | Thick liquid | - |
| **5** | Dodecyl sulfate | Thick liquid | - |
| **6** | Palmitic acid | Thick liquid | - |
| **7** | Isobutyric acid | Thick liquid | - |
| **8** | 1,5-Naphthalene disulfonic acid | Solid | Crystallized in DCM-DIPE and CHCl₃-Hexane |
| **9** | Capric acid | Thick liquid | - |
| **10** | L-pyroglutamic acid | Solid | - |
| **11** | (1 S)-(+)-Camphor-10-sulfonic acid | Solid | - |
| **12** | Nicotonic acid | Solid | - |
| **13** | Mucic acid | Solid | - |
| **14** | Caproic acid | Thick liquid | - |
| **15** | Dichloroacetic acid | Thick liquid | - |
| **16** | Benzenesulfonic acid | Solid | Crystallized in IPA/191-194 °C |
| **17** | P-toluenesulfonic acid | Solid | Amorphous |
| **18** | Methanesulfonic acid | Solid | Crude |
| **19** | Maleic acid | Solid | Amorphous/ 93-95 °C |
| **20** | Acetic acid | Thick liquid | - |
| **21** | Sulfuric acid | Thick liquid | - |
| **22** | Di-benzoyl-L-tartaric acid | Solid | Crystallized in Toluene-DIPE, CHCl₃-DIPE/ 108-110 °C |
| **23** | D(-)-Mandelic acid | Solid | - |
| **24** | Phosphoric acid | solid | Crystallized in ethylacetate/ 225-245°C |
| **25** | Nitric acid | Solid | Crystallized in IPA |
| **26** | Hydrobromic acid | Solid | Crystallized in IPA/ 235-240°C |
| **27** | Malic acid | Solid | Amorphous |
| **28** | Citric acid | Solid | Amorphous/ 65-67 °C |
| **29** | L-ascorbic acid | Solid | Amorphous/ > 250 °C |

### Example 52

### Preparation of amorphous (+)-(15,3'R)-quinuclidin-3'-yl 1-phenyl-1,2,3,4-tetrahydro-isoquinoline-2-carboxylate oxalate salt

To a dry, 25 mL single neck round bottom flask dichloromethane (5 mL), (+)-(1 S,3'R)-quinuclidin-3'-yl 1-phenyl-1,2,3,4-tetrahydro-isoquinoline-2-carboxylate oxalate salt (0.5 g) were taken. It was stirred for 5-10 min. to get clear solution. Distilled out the solvent at reduced pressure below 30 °C. Solid oxalate salt was obtained (Wt.-0.49 g, % Purity by HPLC-99.3 %, % Chiral purity-99.8 %). M. p.: 200- 225 °C
XRD shows that compound is amorphous in nature. (Fig. 15)

### Example 53

### Preparation of amorphous (+)-(1S,3'R)-quinuclidin-3'-yl 1-phenyl-1,2,3,4-tetrahydro-isoquinoline-2-carboxylate gentisate salt

To a dry, 25 mL single neck round bottom flask acetone (5 mL), (+)-(1S,3'R)-quinuclidin-3'-yl 1-phenyl-1,2,3,4-tetrahydro-isoquinoline-2-carboxylate gentisate salt obtained in Ex. No. 24 (0.5 g, ) were taken. It was stirred for 5-10 min. to get clear solution. Distilled out the solvent at reduced pressure below 30 °C. Solid gentisate salt was obtained (Wt.- 0.48 g, % Purity by HPLC-99.8 %, % Chiral purity-100 %).
XRD shows that compound is amorphous in nature. (Fig. 16)

### Example 54

### Preparation of (+)-(15,3'R)-quinuclidin-3'-yl 1-phenyl-1,2,3,4-tetrahydroisoquinoline-2-carboxylate gentisate salt

The Solifenacin Di-p-toluoyl-L-tartaric acid salt (18 g, % purity - 99.4 and % chiral purity - 99.3 %) was added to water (200 mL) and cooled to 0-10 °C. To the mixtrure sodium bicarbonate (20 g) dissolved in water was added dropwise at 5-15 °C over a period of 30-60 min.. It was stirred for 1.5-2 h. It was extracted with toluene. Combined all the extracts and washed with water. Organic layer was collected and dried over anhyd, sodium sulfate. It was concentrated at reduced pressure to obtain Solifenacin base
(Wt.- 6.2 g, % Purity-99.2, % Chiral purity-99.3 %).

Solifenacin base (1.0 g) was dissolved in a ethanol (10 mL). To the solution gentisic acid (0.425 g) was added. It was warmed and stirred for 30-60 min. Distilled out the solvent at reduced pressure on Buchi rotavapour. To the semi-solid mass dry toluene was added and subsequently distilled out. Again dry toluene was added and distilled out to obtain the solid salt.

The salt was dissolved in ethyl acetate at reflux temperature. It was cooled to 25-30 °C. It was stirred for 2-3 h. Solid salt was precipitated. It was filtered and washed with cold ethyl acetate and dried to obtain the solid salt (Wt.- 0.950 g, % purity- 99.9, % chiral purity-100 %). M. p.: 169-172 °C.

### Example 55

### Preparation of (+)-(1S,3'R)-quinuclidin-3'-yl 1-phenyl-1,2,3,4-tetrahydroisoquinoline-2-carboxylate besylate salt.

The Solifenacin Di-p-toluoyl-L-tartaric acid salt (18 g, % purity - 99.4 and % chiral purity - 99.3 %) was added to water and cooled to 0-10 °C. To the mixture sodium bicarbonate (20 g) dissolved in water was added dropwise at 5-15 °C over a period of 30-60 min.. It was stirred for 1.5-2 h. It was extracted with toluene. Combined all the extracts and washed with water. Organic layer was collected and dried over anhyd. sodium sulfate. It was concentrated at reduced pressure to obtain Solifenacin base (Wt.- 6.2 g, % Purity-99.2, % Chiral purity-99.3 %).

Solifenacin base (1.0 g) was dissolved in a ethanol. To the solution benzenesulfonic acid (0.436 g) was added. It was warmed and stirred for 30-60 min. Distilled out the solvent at reduced pressure on Buchi rotavapour. To the semi-solid mass dry toluene was added and subsequently distilled out. Again dry toluene was added and distilled out to obtain the solid salt.

The salt was dissolved in IPA (1.5 mL) at reflux temperature. It was cooled to 25-30 °C. It was stirred for 30-60 min.. Solid salt was precipitated. It was filtered and washed with cold IPA and.dried to obtain the solid salt (Wt.- 0.850 g, % purity- 99.7, % chiral purity-100 %). M. p.: 189-191 °C.

### Example 56

### Preparation of Amorphous (+)-(1S,3'R)-quinuclidin-3'-yl 1-phenyl-1,2,3,4-tetrahydro-isoquinoline-2-carboxylate hydrochloride salt

To a dry, 25 mL single neck round bottom flask methanol (4 mL), (+)-(1S,3'R)-quinuclidin-3'-yl 1-phenyl-1,2,3,4-tetrahydro-isoquinoline-2-carboxylate hydrochloride salt (0.4g) were taken. It was stirred, for 5-10 min. to get clear solution. Distilled out the solvent at reduced pressure below 30 °C. Solid hydrochloride salt was obtained (Wt.- 0.4 g , % Purity by HPLC-99.1 %, % Chiral purity-100 %).
XRD(**Fig.-17**) shows that compound is amorphous in nature.

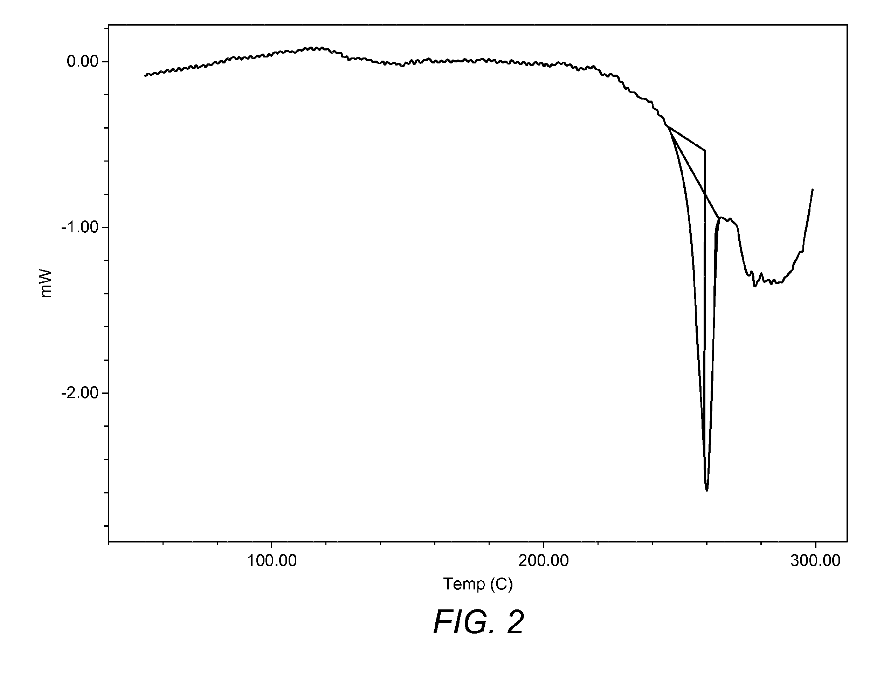

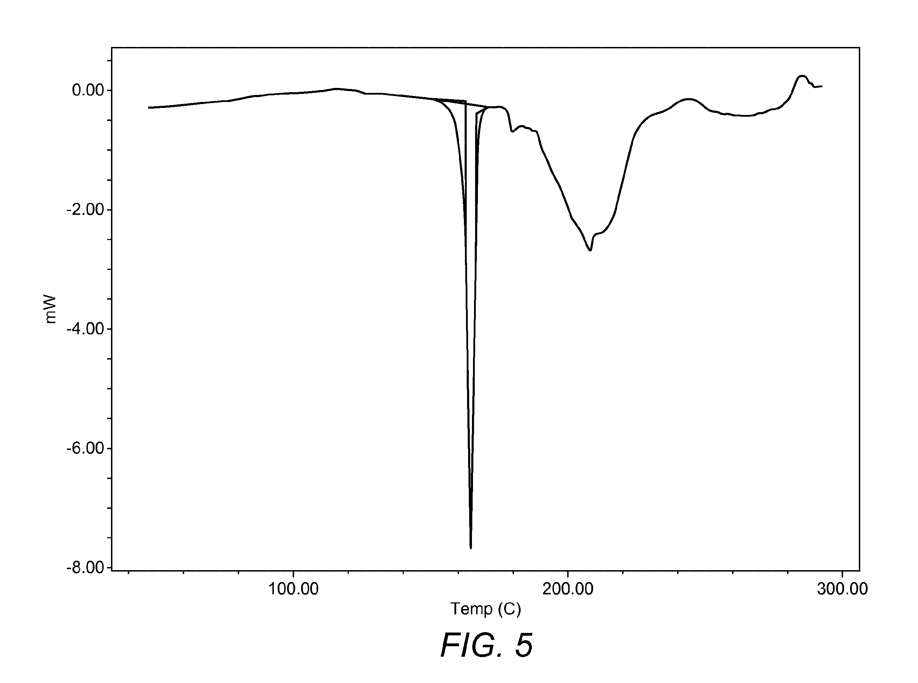

Other embodiments of the invention are as follows:
1. A composition essentially consisting of a salt of Solifenacin obtained by a process wherein, an alkali metal salt of 3(R)-Quinuclidinol is reacted with alkyl (S)-1-phenyl-1,2-3,4-tetrahydroisoquinoline-2-carboxylate to obtain a composition comprising the base of Solifenacin and
   (b) the composition comprising the base of Solifenacin is converted into the salt by reaction with a suitable acid thereby obtaining the composition of the salt, wherein the composition has a purity of at least 98 %, based on the weight of the composition.
2. The composition of paragraph 1 wherein the process further comprises the step of reacting a composition of Solifenacin base with chiral acid to obtain diastereomeric salt of Solifenacin.
3. The composition according to paragraph 2, wherein the diastereomeric salt of Solifenacin is further basified to obtain Solifenacin base, and wherein the Solifenacin base is reacted with a suitable acid to obtain the corresponding chirally pure Solifenacin salt.
4. The composition according to any one of the preceding paragraphs, wherein the composition of salt of Solifenacin is further purified by crystallization and/or recrystallization.
5. The composition of paragraph 1, wherein the salts of Solifenacin are selected from hydrochloride, oxalate, succinate, gentisate, citrate, hydrobromide, sulphate, nitrate, phosphate, maleate, methane sulphonate, ethane sulphonate, benzene sulphonate, tosylate, α-ketoglutarate, glutarate, nicotinate, malate, 1,5-naphthalene disulfonate and ascorbate salts.
6. Salts of Solifenacin, selected from gentisate, citrate, hydrobromide, sulphate, nitrate, phosphate, maleate, methane sulphonate, ethane sulphonate, benzene sulphonate, tosylate, α-ketoglutarate, glutarate, nicotinate, malate, 1,5-naphthalene disulfonate and ascorbate salts.
7. Solifenacin gentisate as defined in paragraph 6, characterized by a PXRD pattern with peaks at about 5.56, 7.06, 7.70, 10.15, 14.63, 15.54, 17.63, 19.4, 19.7, 20.08, 20.68, 21.58, 25.48° ± 0.2° (2θ).
8. Solifenacin gentisate as defined in paragraph 6, characterized by a PXRD pattern as depicted in Figure 7.
9. Solifenacin gentisate as defined in paragraph 6, which is amorphous characterized by X-ray diffraction pattern as depicted in Figure. 16.
10. Solifenacin besylate as defined in paragraph 6, characterized by a PXRD pattern with peaks at about 5.14, 7.69, 10.27, 12.58, 14.76, 16.05, 16.66, 17.08, 17.46, 18.92, 20.46, 20.93, 21.74, 22.02, 23.22, 24.13, 24.39, 27.28, 28.18)° ± 0.2° two-theta (2θ).
11. Solifenacin besylate as defined in paragraph 6, characterized by a PXRD pattern as depicted in Figure 11.
12. Solifenacin phosphate as defined in paragraph 6, characterized by a PXRD pattern with peaks at about 3.99, 11.22, 12.07, 13.96, 14.96, 16.15, 16.42, 17.51, 17.96, 18.43, 19.2, 19.58, 20.2, 21.31, 22.53, 23.83, 24.94, 25.29, 26.10, 26.46, 26.82, 28.43, 29.74° ± 0.2 ° degrees (2θ).
13. Solifenacin phosphate as defined in paragraph 6, characterized by a PXRD pattern as depicted in Figure 12.
14. Solifenacin hydrobromide as defined in paragraph 6, characterized by a PXRD pattern with peaks at about 8.2, 9.4, 11.98, 13.68, 14.3, 15.27, 15.69, 16.49, 16.77,18.93, 19.29, 19.62, 19.91, 21.04, 21.58, 22.46, 23.14, 24.64, 25.44, 25.78, 27.94, 28.98, 29.43, 30.88, 31.24, 32.38, 33.48, 34.16, 34.43° ± 0.2° degrees (2θ).
15. Solifenacin hydrobromide as defined in paragraph 6, characterized by a PXRD pattern as depicted in Figure 13.
16. Solifenacin 1,5-naphthalene disulfonate as defined in paragraph 6, characterized by a PXRD pattern with peaks at about 5.22, 9.33, 10.28, 11.84, 14.16, 14.84, 15.42, 15.78, 17.18, 17.65, 18.16, 19.06, 19.96, 21.12, 21.63, 21.92, 23.55, 23.80, 26.02, 28.4, 30.35° + 0.2° degrees (2θ).
17. Solifenacin 1,5-Naphthalene disulfonate as defined in paragraph 6, characterized by a PXRD pattern as depicted in Figure 14.
18. Di-paratoluoyl-L-tartaric acid salt of (+)-(1S,3'R)-quinuclidin-3'-yl 1-phenyl-1,2,3,4-tetrahydro-isoquinoline-2-carboxylate
19. Di-paratoluoyl-L-tartaric acid salt of Solifenacin as defined in paragraph 18, characterized by a PXRD pattern with peaks at about 5.67, 11.49, 12.82, 13.49, 13.97, 15.01, 15.68, 16.00, 17.80, 18.28, 19.10, 20.38, 22.36, 23.08, 23.94, 24.50, 24.94 ° ± 2 ° (2θ).
20. Crystalline polymorph of Solifenacin hydrochloride, which is characterized by a PXRD pattern with peaks at about 9.61, 13.18, 14.02, 14.39, 15.58, 15.89, 17.0, 18.94, 19.18, 19.78, 20.98, 21.61 and 26.12 ° ± 0.2° (2θ).
21. Solifenacin hydrochloride as defined in paragraph 20, characterized by a PXRD pattern as depicted in Figure 1.
22. Solifenacin hydrochloride as defined in paragraph 20, characterized by a DSC as depicted in Figure 2.
23. Amorphous Solifenacin hydrochloride.
24. Amorphous Solifenacin hydrochloride as defined in paragraph 23, which is further characterized by X-ray diffraction pattern substantially as depicted in Figure 17.
25. Crystalline polymorph of Solifenacin oxalate, which is characterized by a PXRD pattern with peaks at about 9.78, 12.23, 12.68, 13.42, 15.44, 18.18, 19.56, 20.58, 21.45 and 25.24 ° + 0.2° (2θ).
26. Solifenacin oxalate as defined in paragraph 25, characterized by a PXRD pattern as depicted in Figure 4.
27. Solifenacin Oxalate as defined in paragraph 26, characterized by a DSC as depicted in Figure 5.
28. Amorphous Solifenacin Oxalate.
29. Amorphous Solifenacin Oxalate as defined in paragraph 28, which is further characterized by X-ray diffraction pattern substantially as depicted in Figure 15.
30. A pharmaceutical composition comprising pharmaceutically acceptable salts of Solifenacin together with a liquid or solid carrier, and suitable excipients, wherein the pharmaceutically acceptable salt of Solifenacin is the product of any one of the preceding paragraphs.
31. The pharmaceutical composition of paragraph 30, wherein the product is selected from the group consisting of Solifenacin hydrochloride, Solifenacin oxalate, Solifenacin phosphate, Solifenacin besylate, Solifenacin gentisate and Solifenacin hydrobromide.

## Claims

1. Salts of Solifenacin selected from citrate, hydrobromide, sulphate, nitrate, phosphate, maleate, methane sulphonate, ethane sulphonate, benzene sulphonate, tosylate, α-ketoglutarate, glutarate, nicotinate, malate, 1,5-naphthalene disulfonate and ascorbate salts.

2. Crystalline polymorph of Solifenacin hydrochloride, which is **characterized by** a PXRD pattern with peaks at about 9.61, 13.18, 14.02, 14.39, 15.58, 15.89, 17.0, 18.94, 19.18, 19.78, 20.98, 21.61 and 26.12 ° ± 0.2° (2θ).

3. Solifenacin hydrochloride as claimed in claim 2, **characterized by** a PXRD pattern as depicted in Figure 1.

4. Solifenacin hydrochloride as claimed in claim 2, **characterized by** a DSC as depicted in Figure 2.

5. Amorphous Solifenacin hydrochloride.

6. Amorphous Solifenacin hydrochloride as claimed in claim 5, which is further **characterized by** X-ray diffraction pattern substantially as depicted in Figure 17.

7. Solifenacin besylate as claimed in claim 1, is **characterized by** a PXRD pattern with peaks at about 5.14, 7.69, 10.27, 12.58, 14.76, 16.05, 16.66, 17.08, 17.46, 18.92, 20.46, 20.93, 21.74, 22.02, 23.22, 24.13, 24.39, 27.28, 28.18° ± 0.2° two-theta (2θ).

8. Solifenacin besylate as claimed in claim 1, **characterized by** a PXRD pattern as depicted in Figure 11.

9. Solifenacin phosphate as claimed in claim 1, **characterized by** a PXRD pattern with peaks at about 3.99, 11.22, 12.07, 13.96, 14.96, 16.15, 16.42, 17.51, 17.96, 18.43, 19.2, 19.58, 20.2, 21.31, 22.53, 23.83, 24.94, 25.29, 26.10, 26.46, 26.82, 28.43, 29.74° ± 0.2 ° degrees (2θ).

10. Solifenacin phosphate as claimed in claim 1, **characterized by** a PXRD pattern as depicted in Figure 12.

11. Solifenacin hydrobromide as claimed in claim 1, **characterized by** a PXRD pattern with peaks at about 8.2, 9.4, 11.98, 13.68, 14.3, 15.27, 15.69, 16.49, 16.77,18.93, 19.29, 19.62, 19.91, 21.04, 21.58, 22.46, 23.14, 24.64, 25.44, 25.78, 27.94, 28.98, 29.43, 30.88, 31.24, 32.38, 33.48, 34.16, 34.43° ± 0.2° degrees (2θ).

12. Solifenacin hydrobromide as claimed in claim 1, **characterized by** a PXRD pattern as depicted in Figure 13.

13. Solifenacin 1,5-naphthalene disulfonate as claimed in claim 1, **characterized by** a PXRD pattern with peaks at about 5.22, 9.33, 10.28, 11.84, 14.16, 14.84, 15.42, 15.78, 17.18, 17.65, 18.16, 19.06, 19.96, 21.12, 21.63, 21.92, 23.55, 23.80, 26.02, 28.4, 30.35° + 0.2° degrees (2θ).

14. Solifenacin 1,5-naphthalene disulfonate as claimed in claim 1, **characterized by** a PXRD pattern as depicted in Figure 14.

15. Di-paratoluoyl-L-tartaric acid salt of (+)-(1S,3'R)-quinuclidin-3'-yl 1-phenyl- 1,2,3,4-tetrahydro-isoquinoline-2-carboxylate.

16. Di-paratoluoyl-L-tartaric acid salt of Solifenacin as claimed in claim 15, **characterized by** a PXRD pattern with peaks at about 5.67, 11.49, 12.82, 13.49, 13.97, 15.01, 15.68, 16.00, 17.80, 18.28, 19.10, 20.38, 22.36, 23.08, 23.94, 24.50, 24.94 ° ± 2 °(2θ).

17. Crystalline polymorph of Solifenacin oxalate, **characterized by** a PXRD pattern with peaks at about 9.78, 12.23, 12.68, 13.42, 15.44, 18.18, 19.56, 20.58, 21.45 and 25.24 ° ± 0.2° (2θ).

18. Solifenacin oxalate as claimed in claim 17, **characterized by** a PXRD pattern as depicted in Figure 4.

19. Solifenacin oxalate as claimed in claim 18, **characterized by** a DSC as depicted in Figure 5.

20. Amorphous Solifenacin oxalate.

21. Amorphous Solifenacin oxalate as claimed in claim 20 which is further **characterized by** X-ray diffraction pattern substantially as depicted in Figure 15.

22. A pharmaceutical composition comprising pharmaceutically acceptable salts of Solifenacin together with a liquid or solid carrier, and suitable excipients, wherein the pharmaceutically acceptable salt of Solifenacin is defined according to any one of the preceding claims.

23. The pharmaceutical composition of claim 22, wherein the Solifenacin salt is selected from the group consisting of Solifenacin hydrochloride, Solifenacin oxalate, Solifenacin phosphate, Solifenacin besylate, and Solifenacin hydrobromide.
